(19)

Europäisches
Patentamt
European
Patent Office
Office européen
des brevets

(11)  **EP 4 215 039 A1**

(12)  **EUROPEAN PATENT APPLICATION**

(43) Date of publication:
**26.07.2023  Bulletin 2023/30**

(21) Application number: **22153142.9**

(22) Date of filing: **25.01.2022**

(51) International Patent Classification (IPC):
**A01H 1/00** *(2006.01)*        **C12N 9/02** *(2006.01)*
**C12N 15/82** *(2006.01)*

(52) Cooperative Patent Classification (CPC):
**A01H 1/127; A01H 1/1255; C12N 9/0069;
C12N 15/8282; C12N 15/8286**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **KWS SAAT SE & Co. KGaA
37574 Einbeck (DE)**

(72) Inventors:
• **Bak, Aurélie
ST. LOUIS, MO 63141 (US)**
• **Stahl, Dietmar
37574 Einbeck (DE)**

• **Stirnweis, Daniel
37085 Göttingen (DE)**
• **Scheuermann, Daniela
37574 Einbeck (DE)**
• **Kessel, Bettina
37574 Einbeck (DE)**

(74) Representative: **Eisenführ Speiser
Patentanwälte Rechtsanwälte PartGmbB
Postfach 31 02 60
80102 München (DE)**

Remarks:
The complete document including Reference
Table(s) and the Sequence Listing(s) can be
downloaded from the EPO website

(54)  **LOX3 GENE MODULATION AND ARMYWORM TOLERANCE**

(57)    The present application provides a new technology to confer or enhance insect resistance and, optionally also resistance to fungal pathogens in plants. In particular, the present invention provides a method for conferring or increasing resistance or tolerance to insect and optionally also to fungal pathogens in maize and oil seed rape (OSR) by targeting the endogenous Lox3 gene. Further provided are resistant or tolerant maize or oil seed rape plants, cells, tissues, organs or seeds, which are obtained or obtainable by the method according to the present invention. Expression constructs and vectors for the different approaches described herein are also provided as well as the use of such constructs and methods to confer or increase insect and optionally fungal resistance in plants.

**EP 4 215 039 A1**

## Description

### Technical Field

The present application provides a new technology to confer or enhance insect resistance and, optionally also resistance to fungal pathogens in plants. In particular, the present invention provides a method for conferring or increasing resistance or tolerance to insect and optionally also to fungal pathogens in maize and oil seed rape (OSR) by targeting the endogenous Lox3 gene. By introducing either a gene silencing construct, a genome editing system or a genome modification, which leads to a targeted knock-down or knock-out of the Lox3 gene endogenous to the plant, a new or increased resistance to insect and, optionally fungal pathogens can be created. Further provided are resistant or tolerant maize or oil seed rape plants, cells, tissues, organs or seeds, which are obtained or obtainable by the method according to the present invention. Expression constructs and vectors for the different approaches described herein are also provided as well as the use of such constructs and methods to confer or increase insect and optionally fungal resistance in plants.

### Background

In nature, there is a large variety of organisms causing disease in plants and/or negatively affecting plant health otherwise. These pathogens can be subdivided into (i) infectious organisms, which include fungi, oomycetes, bacteria, viruses, viroids, virus-like organisms, phytoplasmas, protozoa, nematodes, and parasitic plants and (ii) ectoparasites, such as insects, mites, vertebrates, and other pests negatively affecting plant health by eating plant tissue.

Monitoring and securing plant health in both natural and cultivated plant populations are paramount tasks for a reliable supply of food products and a large number of commodities on a global scale. Taken together, pests and diseases in plants cause up to 40 % yield losses every year. Accordingly, there are numerous examples throughout history demonstrating the severe effects of plant disease on society.

For instance, the Great Famine of Ireland from 1845 to 1852 with a total number of deaths of approximately 1 million and the Highland potato famine from 1846 to 1856. The proximate cause of these famines was potato blight, a serious potato and tomato disease caused by the oomycete *Phytophthora infestans.*

From 2010 to 2011, 750,000 hectares of trees in the western Unites States were lost to an infestation by the mountain pine beetle. This infestation was (at least in part) driven by droughts as insects generally thrive in a warmer climate. Droughts also make plant tissue even more nutritious for insects since the lack of water concentrates the contained amino acids. Accordingly, the threat of insects on agricultural crops increases as global warming proceeds.

Insects are among the most relevant organisms attacking and causing damage to agricultural and horticultural crops. The damage they cause is two-fold, namely (i) the direct injuries they cause to a plant for instance by eating plant tissue, and (ii) the indirect injuries caused by fungal, bacterial, or viral infections they transmit. Currently, a global average of 15 % of crops is exclusively lost to insects. At the same time, crop yields need to be increased by at least 40 % in order to be able to reliably feed a population of approximately 9 billion people projected to inhabit the Earth in 2050.

Within the class of insects, a particularly relevant species causing harm to plants is the fall armyworm *(Spodoptera frugiperda)* within in the order *Lepidoptera,* which is the larval life stage of a fall armyworm moth. It shows large-scale invasive behaviour and it is regarded as a pest that can damage and even destroy a wide variety of crops causing large economic damage. It is one of the most damaging pests in corn *(Zea mays).* Geographically, the fall armyworm is distributed in eastern and central North America and in South America. Due to its susceptibility to colder temperatures it can only survive the winter in the southern-most regions of the United States, namely Texas and Florida. However, seasonally it will spread across the eastern United States and up to southern Canada. A CLIMEX model of its potential global distribution indicated much of the potential distribution range in Europe, South Africa, China and Australia. In recent years, the fall armyworm has already been found in at least 28 African countries (e.g. in Ghana, Togo, Benin, Nigeria, and Sao Tome), in Asia (e.g. in the Chinese province of Yunnan and at least 25 other Chinese provinces, in India, in Sri Lanka, and in Bangladesh), and across the Australian continent (e.g. in Western Australia, Queensland, the Northern Territory, New South Wales, North Queensland, and the Torres Strait Islands).

The fall armyworm's global distribution bears the potential for further severe economic damage. For example, it caused significant damage to maize crops in Africa in 2016. Moreover, heavy infestation of fall armyworm was reported for plantations in Sri Lanka and has reached China's northeastern corn belt in 2020 and China's Ministry of Agriculture and Rural Affairs has rated the situation as 'very grave'. The fall armyworm is also expected to severely impact Australia's wool industry as it feeds on all major grazing plants.

Another extremely relevant species causing major damage to plants is the corn leafhopper *(Dalbulus maidis)* within the order *Hemiptera.* This species is widely spread through most tropical and subtropical regions on earth including Southeast Asia and China, Australia, Africa, and both North and South America. The corn leafhopper is predominantly a pest of maize and its relatives with high economic importance. In addition to the direct damage caused by its herbivorous lifestyle, it also functions as a vector for several species-specific maize viruses, such as maize stripe virus (MSV), maize mosaic virus (MMV), and maize tenuivirus (MStV). The latter two are pathogenic viruses, which might reduce crop yields by 9 to 90 %. It has even been suggested that the spreading of *Dalbulus maidis* together with MMV and MStV to the New World contributed to the collapse of the Mayan civilization. Infestation with *Dalbulus maidis* will lead to physical damage the plant as the insect breaks through the vascular tissue of the plant in order to feed on the exuding sap. Eventually, this damage will cause yellowing of leaves, wilting, stem weakness, and finally death. The feeding behaviour alone of *Dalbulus maidis* might cause 10 to 15 % crop loss.

Another exemplary insect species of major importance for plant damage is the globally distributed green peach aphid *(Myzus persicae),* which is the most significant aphid pest of peach trees and is known to attack more than 240 plant species from 64 different families. As a result of an infestation by the green peach aphid, peach trees exhibit decreased growth, shrivelling of the leaves and death of various tissues. Prolonged infestation can lead to a drastic decrease in yield of various root and foliage crops. Furthermore, the green peach aphid can be a serious pest problem for oil seed rape crop. For example, a drastic infestation of oil seed rape by the green peach aphid in the south east of Romania was reported in autumn 2018. In this instance, a high pest density of 243 aphids per oil seed rape leaf was reported. Moreover, the green peach aphid acts as a vector for plant viruses, such as pepper potyviruses, potato virus Y (PVY), tobacco etch virus (TEV), and cucumber mosaic virus (CMV), which can be passed on to many different food crops.

The green belly stink bug *(Dichelops melacanthus)* is a key pest in corn and wheat, two of the most important crop plants on a global scale. It is distributed in nearly all of South America and attacks at least 29 plant species. Besides corn and wheat, other affected crops of major economic importance include soybean, oats, and triticale. In the early life stages of plants, the damage caused by *Dichelops melacanthus* can be particularly severe as the insect physically damages the vascular tissue in order to feed on sap and thereby possibly introduces salivary toxic enzymes into the stem base of the plants. Eventually, this leads to withering of leaves, wilting, and finally to death of the plant.

Fungi and fungi-like organisms constitute the largest number of plant pathogens and are responsible for a wide range of plant diseases, which have been reported to lead up to 100 % crop loss. For instance, most vegetable diseases are due to fungal infections. Causes for fungal infections include spreading through water and wind and through contaminated soils, animals, seedlings, and other plant materials. Fungi enter plants through naturally occurring openings, such as stomata and wounds caused by e.g. pruning, harvesting, insects, hail, and other causes for mechanical damage.

*Leptosphaeria maculans* (anamorph: *Phoma lingam*) is a globally distributed fungal pathogen of the phylum Ascomycota, which is the causal agent of phoma stem canker or blackleg disease in *Brassica* crops. The fungus can directly penetrate plant roots. Symptoms of blackleg disease include basal stem cankers, small grey lesions on leaves, and root rot. Basal stem cankers are the main cause for drastic crop yield losses. *Leptosphaeria maculans* infects a variety of different *Brassica* species including oilseed rape *(Brassica napus)* and cabbage (*Brassica oleracea*). It is especially virulent in oilseed rape. Infections can lead to decrease in crop yields by about 10 to 20 %. The release of ascospores by *Leptosphaeria maculans* typically occurs from September to November at moderate temperatures between 8 °C and 15 °C and a relatively high humidity. During this time, agricultural oilseed rape is the most vulnerable.

Another economically relevant fungal plant pathogen is the soil-borne *Plasmodiophora brassicae,* which belongs to the group Phytomyxea and causes clubroot in a large number of plants from the family *Brassicaceae.* Symptoms of clubroot include gall formation and distortion on latent roots giving rise to the shape of a club or spindle. In cabbages, these effects on the roots lead to underdeveloped heads or even an overall failure to head at all, which is often followed by decline in vigor and death of the plant. Other symptoms include wilting, yellowing and stunted growth. In the late 19th century, severe epidemics of clubroot lead to the loss of major parts of cabbage crops in St. Petersburg. Presently, clubroot is still a disease of great economic relevance affecting approximately 10% of cultured areas. Clubroot infections can affect entire fields and thus significantly reduce crop yields and even result in no crop yield at all. On the field, the pathogen can survive for years as resting spores.

Hence, there is an urgent and rapidly growing demand for efficient strategies to minimize and confine the economic damage caused by plant pathogens.

The most commonly used form of protection against insects are different insecticides. In southern regions, insecticides have to be applied every day to corn in order to be able to manage fall armyworm infestation. In 2020, a biopesticide - namely a caterpillar-specific virus - was approved under emergency regulations in Australia in order to control the fall armyworm. Different Parasitoids (e.g. the wasp *Trichogramma pretiosum*) are also used. The use of insecticides has many disadvantages. For instance, many insecticides non-selectively harm or even kill other species in addition to the targeted ones. A prominent example of this phenomenon is the observed decline of pollinators, such as bees due to colony collapse disorder (CCD). Even sub-lethal amounts of insecticides can affect bee foraging behaviour. The loss of pollinators results in a reduction of crop yields. Besides that, birds may be killed when consuming plants or insects that were in contact with insecticides. Populations of insectivorous birds also decline due to the collapse of their prey populations. Especially the spraying of insecticides on corn and wheat in Europe is believed to have caused a decline in flying insects of about 80 %, which in turn has reduced the European bird population by one to two thirds.

Runoff and percolation of (improperly applied) insecticides can negatively affect the quality of water sources and harm the natural ecology, which has an indirect effect on human populations through biomagnification and bioaccumulation.

Alternatively, in order to manage insect infestation different agricultural techniques, such as e.g. planting early, avoiding staggered planting, and inter-cropping are applied. These strategies are extremely cost- and time-intensive, cannot universally be applied due to ecological limitations and thus bear several risks. For example, inter-cropping has successfully been applied against fall army worm infestations in small-scale greenhouse, garden and field experiments. However, at larger commercial scales the pest damage to only a very small portion of crop plants could be reduced using this method.

Fungal plant diseases may be controlled employing fungicides and various other agricultural techniques. Typically applied fungicides include EBI and MBC fungicides, which can decrease instances of disease in crop populations. However, the use of fungicides bears similar disadvantages as described above for insecticides. Additionally, some fungicides also negatively affect the overall growth of the crop plants. Cultural methods include stubble and crop rotation. Removing stubble has been shown to decrease the risk of *Leptosphaeria maculans* infection in *Brassica* species. In canola crops, crop rotation has been shown to reduce blackleg.

Based on the difficulties and disadvantages of agricultural and chemical methods for reducing infestations and infections, considerable effort is put into genetically engineering and optimizing the crop plants themselves in order to make them more tolerant towards environmental conditions and pests.

In case of maize and other crop plants, the lipoxygenase (Lox) pathway is known to play a role regarding resistance to pathogens. However, previous studies show that the actual function of the Lox pathway with respect to resistance towards certain pathogens is highly unpredictable due to an extremely high degree of host- and pathogen specificity to the extent that within the same host species, Lox-derived metabolism can even have completely opposite effects on pathogen resistance in a pathogen species-specific manner.

In the Lox pathway, various highly specialized forms of lipoxygenases catalyse the synthesis of hydroperoxy polyunsaturated fatty acids, which are substrates to at least seven different enzyme families. For example, plant oxylipins produced via the Lox pathway have been demonstrated to function as environmentally and developmentally regulated defence- and development signals. It has also been demonstrated that these molecules function as powerful regulators of sporogenesis and mycotoxin biosynthesis in fungi. Specifically, it could be demonstrated that fatty acid hydroperoxides derived from 9-Lox induce conidiation and mycotoxin production.

In 2007, Gao et al. (MPMI, vol. 20, No. 8, 2007, pp. 922-933) generated maize mutants lacking functional 9-Lox by disruption of *ZmLox3* (gene coding for 9-Lox) via transposon mutagenesis. Accordingly, decreased levels of 9-Lox-derived hydroperoxides were observed. Consequently, in kernels of the *lox3* mutants reduced conidiation and reduced production of mycotoxin fumonisin B1 by *Fusarium verticillioides* were observed as compared to the wild type. Additionally, lox3 mutants demonstrated reduced disease severity of various fungi-derived diseases, such as anthracnose leaf blight *(Colletotrichum graminicola),* southern leaf blight (*Cochliobolus heterostrophus*), and stalk rots (*Fusarium verticillioides* and *Colletotrichum graminicola*). They conclude from these findings that 9-LOX-based metabolism apparently is required for fungal pathogenesis, including disease development and production of spores and mycotoxins.

However, in 2008, Gao et al. (MPMI, vol. 21, No. 1, 2008, pp. 98-109) demonstrated that *lox3-4* knockout mutants of maize displayed increased attractiveness to root-knot nematodes (*Meloidogyne incognita*). They observed that in these *lox3-4* knockout mutants a phenylalanine ammonia lyase (PAL) gene was not inducible in a root-knot nematode-dependent manner. This suggests a PAL-mediated metabolism to be of importance for root-knot nematode resistance. Moreover, in *lox3-4* knockout mutants a root-specific increase in jasmonic acid, ethylene, and salicylic acid and overexpression of the respective biosynthetic genes was observed. Therefore, the ZmLox3-mediated metabolic pathway apparently is required for the three major defence signalling pathways for the resistance against nematodes.

Therefore, there is a great need in defining new molecular mechanisms to increase tolerance or resistance of major crop plants towards their cognate and specific pathogens, by specifically studying and controlling endogenous signalling pathways to optimize plant defence against pathogens and thus yields. By defining new ways to influence and control Lox3-pathways in different crop plants in response to some specific insect and fungal pathogens triggering a specific response in the respective target plant, these objects could be achieved by the methods as presented and disclosed below.

## Summary of the Invention

In one aspect, the present invention relates to a method for conferring or increasing resistance or tolerance to fall armyworm *(Spodoptera frugiperda)* and, optionally to a fungal pathogen, in particular *Fusarium species,* to/in a plant, preferably a maize *(Zea mays)* plant, comprising the steps of:

    (i) providing at least one plant cell, preferably a maize plant cell;

(ii) introducing into the at least one plant cell at least one gene silencing construct, at least one genome editing system or a genome modification, which leads to a targeted knock-down or a knock-out of a Lox3 gene endogenous to the plant;

(iii) obtaining at least one modified plant cell having reduced or abolished expression of the Lox3 gene; and

(iv) obtaining at least one plant cell, tissue, organ, plant or seed having reduced or abolished expression of the Lox3 gene, optionally after an additional step of regenerating the plant tissue, organ, plant or seed from the at least one modified cell.

In one embodiment of the method described above, the Lox3 gene is represented by a nucleic acid sequence of SEQ ID NO: 6, 7, 9, 10, 12, 13, 15, 16, 87 or 88, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 6, 7, 9, 10, 12, 13, 15, 16, 87 or 88, respectively.

In another embodiment of the method described above, the Lox3 gene encodes an amino acid sequence of SEQ ID NO: 8, 11, 14, 17 or 89 or an amino acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 8, 11, 14, 17 or 89.

In one embodiment of the method according to any of the embodiment described above, in step (ii) a construct is introduced into the at least one plant cell, which targets the Lox3 gene for gene silencing wherein the construct is or wherein the construct encodes an RNAi construct comprising a sense and an antisense sequence targeting the Lox3 gene, the RNAi construct forming an RNA hairpin upon transcription,

(a) wherein the sense sequence is encoded by a nucleic acid sequence of SEQ ID NO: 1, or a nucleic acid sequence having a sequence identity of at least 90%, 91%, 92%, 93%, 94%, 95%, 96%, 97%, 98% or at least 99% to the sequence of SEQ ID NO: 1; and/or

(b) wherein the antisense sequence is encoded by a nucleic acid sequence of SEQ ID NO: 2, or a nucleic acid sequence having a sequence identity of at least 90%, 91%, 92%, 93%, 94%, 95%, 96%, 97%, 98% or at least 99% to the sequence of SEQ ID NO: 2, and/or

(c) preferably wherein the construct comprises a nucleic acid sequence of SEQ ID NO: 4, or a nucleic acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4, optionally wherein the construct is introduced into the at least one plant cell by transformation or transfection mediated by biolistic bombardment, *Agrobacterium-mediated* transformation, micro- or nanoparticle delivery, chemical transfection, or a combination thereof.

In one embodiment of the method described above, the RNA hairpin has an intervening intron loop sequence comprising a nucleic acid sequence of SEQ ID NO: 3, or a nucleic acid sequence having a sequence identity of at least 85%, 86%, 87%, 88%, 89%, 90%, 91%, 92%, 93%, 94%, 95%, 96%, 97%, 98% or at least 99% to the sequence of SEQ ID NO: 3.

In another embodiment of the method described above, in step (ii) a construct is introduced into the at least one plant cell, which targets the Lox3 gene for gene silencing, preferably wherein the construct is introduced as part of a vector the vector being introduced into the plant cell, which vector comprises or consists of a nucleic acid sequence of SEQ ID NO: 5, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 5.

In one embodiment of the method described above, in step (ii) at least one genome editing system is introduced into the at least one cell, which construct targets the Lox3 gene, wherein the at least one genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same,

optionally, wherein the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or wherein the at least one genome editing system is selected from a zinc finger nuclease system, or a transcription activator-like nuclease system, or a meganuclease system, or any combination, variant, or an active fragment thereof; and optionally, wherein the at least one genome editing system is introduced into the at least one maize cell by transformation or transfection mediated by biolistic bombardment, Agrobacterium-mediated transformation, micro- or nanoparticle delivery, chemical transfection, or a combination thereof.

In another embodiment of the method described above, in step (ii) a mutagenesis is performed to obtain at least one genome modification on a single or on a plurality of cell(s) by applying chemicals or radiation, preferably wherein an alkylating agent, including ethyl methanesulfonate is applied to the single or the plurality of cell(s) to induce mutagenesis.

In a further embodiment of the method described above, one or more mutations in the Lox3 gene are inserted and identified by TILLING in step (ii) and/or one or more cell(s) with knock-down or knock-out mutations in the Lox3 gene are selected in step (ii).

In one aspect, the present invention relates to a maize cell, maize tissue, maize organ, maize plant or maize seed obtained or obtainable by a method according to any of the embodiments described above.

In yet another aspect, the present invention relates to an expression construct, which targets the Lox3 gene in maize for gene silencing, wherein the construct encodes an RNAi construct comprising a sense and an antisense sequence targeting the Lox3 gene endogenous to a maize plant, which RNAi construct forms an RNA hairpin upon transcription, wherein

(a) the sense sequence is encoded by a nucleic acid sequence of SEQ ID NO: 1, or a nucleic acid sequence having a sequence identity of at least 90%, 91%, 92%, 93%, 94%, 95%, 96%, 97%, 98% or at least 99% to the sequence of SEQ ID NO: 1; and/or

(b) wherein the antisense sequence is encoded by a nucleic acid sequence of SEQ ID NO: 2, or a nucleic acid sequence having a sequence identity of at least 90%, 91%, 92%, 93%, 94%, 95%, 96%, 97%, 98% or at least 99% to the sequence of SEQ ID NO: 2, and/or optionally wherein the RNA hairpin has an intervening intron loop sequence comprising a nucleic acid sequence of SEQ ID NO: 3, or a nucleic acid sequence having a sequence identity of at least 85%, 86%, 87%, 88%, 89%, 90%, 91%, 92%, 93%, 94%, 95%, 96%, 97%, 98% or at least 95% to the sequence of SEQ ID NO: 3; and/or

(c) preferably wherein the construct comprises a nucleic acid sequence of SEQ ID NO: 4, or a nucleic acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4.

In another aspect, the present invention relates to an RNAi hairpin construct for conferring or increasing resistance or tolerance to fall armyworm *(Spodoptera frugiperda)* and, optionally to a fungal pathogen, in particular *Fusarium species,* to/in a maize plant *(Zea mays),* wherein the RNAi hairpin construct comprises a nucleic acid sequence of SEQ ID NO: 4, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4.

In one aspect, the present invention relates to an expression construct encoding a genome editing system, which targets the Lox3 gene in maize, wherein the genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same,

preferably wherein the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or wherein the at least one genome editing system is selected from a zinc finger nuclease system, or a transcription activator-like nuclease system, or a meganuclease system, or any combination, variant, or an active fragment thereof.

In yet another aspect, the present invention relates to a maize cell, maize tissue, maize organ, maize plant or maize seed comprising an expression construct, or a vector encoding the same, or an RNAi hairpin construct, or a vector encoding the same, according to any of the embodiments described above.

In a further embodiment, the present invention relates to a use of at least one gene silencing construct and/or of at least one genome editing system and/or of at least one genome modification as defined in any of the embodiments described above, which leads to a targeted knock-down or a knock-out of the endogenous a Lox3 gene, for conferring or increasing resistance or tolerance to fall armyworm (Spodoptera frugiperda) and, optionally to a fungal pathogen, in particular Fusarium species, to/in a plant, preferably a maize (Zea mays) plant.

In one aspect, the present invention relates to a method for conferring or increasing resistance or tolerance to an insect and, optionally a fungal pathogen to/in a plant comprising the steps of:

(i) providing at least one plant cell;

(ii) introducing into the at least one plant cell at least one gene silencing construct, at least one genome editing system or a genome modification, which leads to a targeted knock-down or a knock-out of a Lox3 gene endogenous to the plant;

(iii) obtaining at least one modified plant cell having reduced or abolished expression of the Lox3 gene; and

(iv) obtaining at least one plant cell, tissue, organ, plant or seed having reduced or abolished expression of the Lox3 gene, optionally after an additional step of regenerating the plant tissue, organ, plant or seed from the at least one modified cell.

In one embodiment of the method described above, the method is for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize (Zea mays).

In another embodiment of the method described above, the method is for conferring or increasing resistance or tolerance to one or more insect(s) selected from the group consisting of green peach aphid *(Myzus persicae),* diamondback moth *(Plutella xylostella),* cabbage stem flea beetle *(Psylliodes chrysocephala),* crucifer flea beetle *(Phyllotreta cruciferae),* striped flea beetle *(Phyllotreta striolata),* hop flea beetle *(Psylliodespunctulata),* rape stem weevil *(Ceutorhynchus picitarsis)* and cabbage stem weevil *(Ceutorhynchus quadridens)* and, optionally conferring or increasing resistance or tolerance to one or more fungal pathogen(s) selected from the group consisting of *Phoma lingam* and *Plasmodiophora brassicae* to/in oilseed rape (Brassica napus).

In one embodiment of the method described above, the *Lox3* gene is represented by a nucleic acid sequence of SEQ ID NO: 6, 7, 9, 10, 12, 13, 15, 16, 87 or 88 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 6, 7, 9, 10, 12, 13, 15, 16, 87 or 88.

In another embodiment of the method described above, the Lox3 gene encodes an amino acid sequence of SEQ ID NO: 8, 11, 14, 17 or 89 or an amino acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 8, 11, 14, 17 or 89.

In a further embodiment of the method described above, the Lox3 gene is represented by a nucleic acid sequence of SEQ ID NO: 75, 76, 77, 78, 83, 84, 85 or 86 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: : 75, 76, 77, 78, 83, 84, 85 or 86.

In yet another embodiment of the method described above, the Lox3 gene encodes an amino acid sequence of SEQ ID NO: 79, 80, 81 or 82 or an amino acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 79, 80, 81 or 82.

In an embodiment of the method described above, in step (ii) a construct is introduced into the at least one plant cell, which targets the Lox3 gene for gene silencing.

In one embodiment of the method described above, the construct is or the construct encodes an RNAi construct comprising a sense and an antisense sequence targeting the Lox3 gene, the RNAi construct forming an RNA hairpin upon transcription.

In one embodiment of the method described above, the method is for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, *optionally* conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis to/in maize (Zea mays) and* the sense sequence is encoded by a nucleic acid sequence of SEQ ID NO: 1, or a nucleic acid sequence having a sequence identity of at least 90%, 91%, 92%, 93%, 94%, 95%, 96%, 97%, 98% or at least 99% to the sequence of SEQ ID NO: 1.

In another embodiment of the method described above, the method is for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays)* and the antisense sequence is encoded by a nucleic acid sequence of SEQ ID NO: 2, or a nucleic acid sequence having a sequence identity of at least 90%, 91%, 92%, 93%, 94%, 95%, 96%, 97%, 98% or at least 99% to the sequence of SEQ ID NO: 2.

In yet another embodiment of the method described above, the method is for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus) and, optionally* conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis to/in maize (Zea mays) and* the RNA hairpin has an intervening intron loop sequence comprising a nucleic acid sequence of SEQ ID NO: 3, or a nucleic acid sequence having a sequence identity of at least 85%, 86%, 87%, 88%, 89%, 90%, 91%, 92%, 93%, 94%, 95%, 96%, 97%, 98% or at least 99% to the sequence of SEQ ID NO: 3.

In a further embodiment of the method described above, the method is for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays)* and the construct comprises a nucleic acid sequence of SEQ ID NO: 4, or a nucleic acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4.

In yet a further embodiment of the method described above, the method is for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays)* and a vector is introduced into the plant cell, which vector comprises or consists of a nucleic acid sequence of SEQ ID NO: 5, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 5.

In one embodiment of the method described above, the construct is introduced into the at least one plant cell by transformation or transfection mediated by biolistic bombardment, *Agrobacterium-mediated* transformation, micro- or nanoparticle delivery, chemical transfection, or a combination thereof.

In a further embodiment of the method described above, in step (ii) at least one genome editing system is introduced into the at least one cell, which targets the Lox3 gene, wherein the at least one genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same.

In one embodiment of the method described above, the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or the at least one genome editing system, is selected from a zinc finger nuclease system, or a transcription activator-like nuclease system, or a meganuclease system, or any combination, variant, or an active fragment thereof.

In another embodiment of the method described above, the at least one genome editing system is introduced into the at least one cell by transformation or transfection mediated by biolistic bombardment, *Agrobacterium-mediated* transformation, micro- or nanoparticle delivery, chemical transfection, or a combination thereof.

In yet another embodiment of the method described above, the method is for *conferring* or increasing resistance or tolerance to one or more insect(s) selected from the group consisting of green peach aphid *(Myzus persicae)*, diamondback moth *(Plutella xylostella)*, cabbage stem flea beetle *(Psylliodes chrysocephala)*, crucifer flea beetle *(Phyllotreta cruciferae)*, striped flea beetle *(Phyllotreta striolata)*, hop flea beetle *(Psylliodes punctulata)*, rape stem weevil *(Ceutorhynchus picitarsis)* and cabbage stem weevil *(Ceutorhynchus quadridens)* *and, optionally* conferring or increasing resistance or tolerance to one or more fungal pathogen(s) selected from the group consisting of *Phoma lingam* and *Plasmodiophora brassicae to/in oilseed rape (Brassica napus), and* the at least one genome editing system comprises a crRNA encoded by a nucleic acid sequence of any of SEQ ID NOs: 46 to 49, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of any of SEQ ID NOs: 46 to 49.

In one embodiment of the method described above, the genome editing system is encoded by a plasmid of the nucleic acid sequence of SEQ ID NO: 50 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 50.

In another embodiment of the method described above, in step (ii) a mutagenesis is performed on a single or on a plurality of cell(s) by applying chemicals or radiation.

In one embodiment of the method described above, an alkylating agent, in particular ethyl methanesulfonate is applied to the single or the plurality of cell(s) to induce mutagenesis.

In another embodiment of the method described above, one or more mutations in the Lox3 gene are inserted and identified by TILLING in step (ii).

In a further embodiment of the method described above, one or more cell(s) with knock-down or knock-out mutations in the Lox3 gene are selected in step (ii).

In another aspect, the present invention relates to a maize cell, maize tissue, maize organ, maize plant or maize seed obtained or obtainable by a method according to any of the embodiments described above.

In a further aspect the present invention relates to an oilseed rape cell, oilseed rape tissue, oilseed rape organ, oilseed rape plant or oilseed rape seed obtained or obtainable by a method according to any of the embodiments described above.

In yet a further aspect, the present invention relates to an expression construct, which targets the Lox3 gene in maize for gene silencing, wherein the construct encodes an RNAi construct comprising a sense and an antisense sequence targeting the Lox3 gene endogenous to a maize plant, which RNAi construct forms an RNA hairpin upon transcription.

In a further aspect, the present invention relates to an expression construct, which targets the Lox3 gene in oilseed rape for gene silencing, wherein the construct encodes an RNAi construct comprising a sense and an antisense sequence targeting the Lox3 gene endogenous to an oilseed rape plant, which RNAi construct forms an RNA hairpin upon transcription.

In one embodiment of the expression construct described above, the sense sequence is encoded by a nucleic acid sequence of SEQ ID NO: 1, or a nucleic acid sequence having a sequence identity of at least 90%, 91%, 92%, 93%, 94%, 95%, 96%, 97%, 98% or at least 99% to the sequence of SEQ ID NO: 1.

In another embodiment of the expression construct described above, the antisense sequence is encoded by a nucleic acid sequence of SEQ ID NO: 2, or a nucleic acid sequence having a sequence identity of at least 90%, 91%, 92%, 93%, 94%, 95%, 96%, 97%, 98% or at least 99% to the sequence of SEQ ID NO: 2.

In yet another embodiment of the expression construct described above, the RNA hairpin has an intervening intron loop sequence comprising a nucleic acid sequence of SEQ ID NO: 3, or a nucleic acid sequence having a sequence identity of at least 85%, 86%, 87%, 88%, 89%, 90%, 91%, 92%, 93%, 94%, 95%, 96%, 97%, 98% or at least 99% to the sequence of SEQ ID NO: 3.

In one embodiment of the expression construct described above, the construct comprises a nucleic acid sequence of SEQ ID NO: 4, or a nucleic acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4.

In another aspect, the present invention relates to a vector comprising or consisting of a nucleic acid sequence of SEQ ID NO: 5 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 5.

In yet another aspect, the present invention relates to an RNAi hairpin construct conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays),* wherein the RNAi hairpin construct comprises a nucleic acid sequence of SEQ ID NO: 4, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4.

In a further aspect, the present invention relates to an expression construct encoding a genome editing system, which targets the Lox3 gene in maize, wherein the genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same.

In another aspect the present invention relates to an expression construct encoding a genome editing system, which targets the Lox3 gene in oilseed rape, wherein the genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same.

In one embodiment of the expression construct described above, the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or the at least one genome editing system is selected from a zinc finger nuclease system, or a transcription activator-like nuclease system, or a meganuclease system, or any combination, variant, or an active fragment thereof.

In another embodiment of the expression construct described above, the expression construct comprises a crRNA encoded by a nucleic acid sequence of any of SEQ ID NOs: 46 to 49, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of any of SEQ ID NO: 46 to 49.

In a further embodiment of the expression construct described above, the genome editing system is encoded by a plasmid of the nucleic acid sequence of SEQ ID NO: 50 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 50.

In another aspect, the present invention relates to a vector encoding an expression construct according to any of the embodiments described above.

In further aspect, the present invention relates to a maize cell, maize tissue, maize organ, maize plant or maize seed comprising an expression construct or a vector according to any of the embodiments described above.

In yet a further aspect, the present invention relates to an oilseed rape cell, oilseed rape tissue, oilseed rape organ, oilseed rape plant or oilseed rape seed comprising an expression construct or a vector according to any of the embodiments described above.

In one aspect the present invention relates to the use of at least one gene silencing construct, at least one genome editing system or a genome modification, which leads to a targeted knock-down or a knock-out of the endogenous a Lox3 gene, for conferring or increasing resistance or tolerance to one or more insect(s) and, optionally one or more fungal pathogen(s) to a plant.

In one aspect the present invention relates to the use of at least one gene silencing construct, at least one genome editing system or a genome modification, which leads to a targeted knock-down or a knock-out of the endogenous a Lox3 gene, for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays).*

In another aspect the present invention relates to the use of at least one gene silencing construct, at least one genome editing system or a genome modification, which leads to a targeted knock-down or a knock-out of the endogenous a Lox3 gene, for conferring or increasing resistance or tolerance to one or more insect(s) selected from the group consisting of green peach aphid *(Myzus persicae),* diamondback moth *(Plutella xylostella)*, cabbage stem flea beetle *(Psylliodes chrysocephala)*, crucifer flea beetle *(Phyllotreta cruciferae),* striped flea beetle *(Phyllotreta striolata),* hop flea beetle *(Psylliodes punctulata)*, rape stem weevil *(Ceutorhynchus picitarsis)* and cabbage stem weevil *(Ceutorhynchus quadridens)* and, optionally conferring or increasing resistance or tolerance to one or more fungal pathogen(s) selected from the group consisting of *Phoma lingam* and *Plasmodiophora brassicae* to/in oilseed rape *(Brassica napus)*.

In a further aspect, the present invention relates to the use of a construct, the construct being or encoding an RNAi construct comprising a sense and an antisense sequence targeting the endogenous Lox3 gene of a maize plant, wherein the RNAi construct forms an RNA hairpin upon transcription, for conferring or increasing resistance or tolerance to one or more insect(s) selected from the group consisting of fall army worm *(Spodoptera frugiperda)*, corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species*, in particular *Colletotrichum graminicola* and *Colletotrichum sublineolum, Diplodia species, Cercospora zeina* and *Cercospora zeae-maydis* to/in maize *(Zea mays)*.

In yet another aspect, the present invention relates to the use of a construct, the construct being or encoding an RNAi construct comprising a sense and an antisense sequence targeting the endogenous Lox3 gene of an oilseed rape plant, wherein the RNAi construct forms an RNA hairpin upon transcription, for conferring or increasing resistance or tolerance to one or more insect(s) selected from the group consisting of green peach aphid *(Myzus persicae),* diamondback moth *(Plutella xylostella),* cabbage stem flea beetle *(Psylliodes chrysocephala)*, crucifer flea beetle *(Phyllotreta cruciferae),* striped flea beetle *(Phyllotreta striolata),* hop flea beetle *(Psylliodes punctulata)*, rape stem weevil *(Ceutorhynchus picitarsis)* and cabbage stem weevil *(Ceutorhynchus quadridens)* and, optionally conferring or increasing resistance or tolerance to one or more fungal pathogen(s) selected from the group consisting of *Phoma lingam* and *Plasmodiophora brassicae* to/in oilseed rape *(Brassica napus)*.

In one embodiment of the use described above the sense sequence is encoded by a nucleic acid sequence of SEQ ID NO: 1, or a nucleic acid sequence having a sequence identity of at least 90%, 91%, 92%, 93%, 94%, 95%, 96%, 97%, 98% or at least 99% to the sequence of SEQ ID NO: 1.

In another embodiment of the use described above, the antisense sequence is encoded by a nucleic acid sequence of SEQ ID NO: 2, or a nucleic acid sequence having a sequence identity of at least 90%, 91%, 92%, 93%, 94%, 95%, 96%, 97%, 98% or at least 99% to the sequence of SEQ ID NO: 2.

In a further embodiment of the use described above, the RNA hairpin has an intervening intron loop sequence comprising a nucleic acid sequence of SEQ ID NO: 3, or a nucleic acid sequence having a sequence identity of at least 85%, 86%, 87%, 88%, 89%, 90%, 91%, 92%, 93%, 94%, 95%, 96%, 97%, 98%, or at least 99% to the sequence of SEQ ID NO: 3.

In one embodiment of the use described above, the construct comprises a nucleic acid sequence of SEQ ID NO: 4, or a nucleic acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4.

In another aspect, the present invention relates to the use of a vector, which vector comprises or consists of a nucleic acid sequence of SEQ ID NO: 5, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 5 for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays)*.

In yet another aspect, the present invention relates to a use of a genome editing system, which targets the endogenous Lox3 gene in a maize plant, wherein the genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same

for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays).*

In a further aspect, the present invention relates to a use of a genome editing system, which targets the endogenous Lox3 gene in an oilseed rape plant, wherein the genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same

for conferring or increasing resistance or tolerance to one or more insect(s) selected from the group consisting of green peach aphid *(Myzus persicae),* diamondback moth *(Plutella xylostella)*, cabbage stem flea beetle *(Psylliodes chrysocephala),* crucifer flea beetle *(Phyllotreta cruciferae),* striped flea beetle *(Phyllotreta striolata),* hop flea beetle *(Psylliodes punctulata),* rape stem weevil *(Ceutorhynchus picitarsis)* and cabbage stem weevil *(Ceutorhynchus quadridens)* and, optionally conferring or increasing resistance or tolerance to one or more fungal pathogen(s) selected from the group consisting of *Phoma lingam* and *Plasmodiophora brassicae* to/in oilseed rape *(Brassica napus).*

In one aspect, the present invention relates to a use of an expression construct encoding a genome editing system, which targets the endogenous Lox3 gene in a maize plant, wherein the genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same,

wherein the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or the at least one genome editing system is selected from a zinc finger nuclease system, or a transcription activator-like nuclease system, or a meganuclease system, or any combination, variant, or an active fragment thereof for conferring or increasing resistance or tolerance to one or more insect(s) selected from the group consisting of fall army worm *(Spodoptera frugiperda),* corn leafhopper *(Dalbulus maidis*) and green belly stink bug *(Dichelops melacanthus)* and, optionally conferring or increasing resistance or tolerance to one or more fungal pathogen(s) selected from the group consisting of *Fusarium species, Colletotrichum species*, in particular *Colletotrichum graminicola* and *Colletotrichum sublineolum, Diplodia species, Cercospora zeina* and *Cercospora zeae-maydis* to/in maize *(Zea mays).*

In another aspect, the present invention relates to a use of an expression construct encoding a genome editing system, which targets the endogenous Lox3 gene in an oilseed rape plant, wherein the genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same,

wherein the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or the at least one genome editing system is selected from a zinc finger nuclease system, or a transcription activator-like nuclease system, or a meganuclease system, or any combination, variant, or an active fragment thereof

for conferring or increasing resistance or tolerance to one or more insect(s) selected from the group consisting of green peach aphid *(Myzus persicae),* diamondback moth *(Plutella xylostella)*, cabbage stem flea beetle *(Psylliodes chrysocephala),* crucifer flea beetle *(Phyllotreta cruciferae),* striped flea beetle *(Phyllotreta striolata),* hop flea beetle *(Psylliodes punctulata*), rape stem weevil *(Ceutorhynchus picitarsis*) and cabbage stem weevil *(Ceutorhynchus quadridens)* and, optionally conferring or increasing resistance or tolerance to one or more fungal pathogen(s) selected from the group consisting of *Phoma lingam* and *Plasmodiophora brassicae* to/in oilseed rape *(Brassica napus).*

In one embodiment of the use described above, the expression construct comprises a crRNA encoded by a nucleic acid sequence of any of SEQ ID NOs: 46 to 49, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of any of SEQ ID NO: 46 to 49.

In another embodiment of the use described above, the genome editing system is encoded by a plasmid of the nucleic acid sequence of SEQ ID NO: 50 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 50.

In a another aspect, the present invention relates to a use of a vector encoding an genome editing system as defined above for conferring or increasing resistance or tolerance to one or more insect(s) selected from the group consisting of fall army worm (*Spodoptera frugiperda*), corn leafhopper *(Dalbulus maidis)* and green belly stink bug (*Dichelops melacanthus*) and, optionally conferring or increasing resistance or tolerance to one or more fungal pathogen(s) selected from the group consisting of *Fusarium species, Colletotrichum species*, in particular *Colletotrichum graminicola* and *Colletotrichum sublineolum*, *Diplodia species*, *Cercospora zeina* and *Cercospora zeae-maydis* to/in maize *(Zea mays).*

In a further aspect, the present invention relates to a use of a vector encoding an genome editing system as defined above for conferring or increasing resistance or tolerance to one or more insect(s) selected from the group consisting of green peach aphid (*Myzus persicae*), diamondback moth (*Plutella xylostella),* cabbage stem flea beetle (*Psylliodes chrysocephala*), crucifer flea beetle *(Phyllotreta cruciferae),* striped flea beetle (*Phyllotreta striolata*), hop flea beetle (*Psylliodes punctulata),* rape stem weevil (*Ceutorhynchus picitarsis)* and cabbage stem weevil *(Ceutorhynchus quadridens)* and, optionally conferring or increasing resistance or tolerance to one or more fungal pathogen(s) selected from the group consisting of *Phoma lingam* and *Plasmodiophora brassicae* to/in oilseed rape *(Brassica napus).*

Brief Description of Sequences

| SEQ ID NO. | Description |
|---|---|
| 1 | RNAi target region sense strand before RGA2intronII |
| 2 | RNAi target region antisense strand after the RGA2intronII |
| 3 | RGA2intronII sequence from *Triticum aestivum*- second intron of RGA2 (resistance gene analog; gene involved in leaf rust resistance in wheat) according to Loutre et al., 2009, The Plant Journal 60, 1043-1054 |
| 4 | Sequence of RNAi cassette (RNAi target region sense strand before RGA2intronII + RGA2intronII + RNAi target region antisense strand after the RGA2intronII) |
| 5 | Sequence of a complete exemplary RNAi vector, here starting with ColE1 ori |
| 6 | Zm00001eb054040_B73AGP05_T001_genomic (ZmLox3) - Genomic sequence of Lox3 from *Zea mays* |
| 7 | Zm00001eb054040_B73AGP05_T001_CDS (ZmLox3) - Coding sequence of Lox3 from *Zea mays* |
| 8 | Zm00001 eb054040_B73AGP05_T001 _pro (ZmLox3) - Amino acid sequence of Lox3 from *Zea mays* |
| 9 | ZmLox3_gDNA A188- genomic sequence of Lox3 from *Zea mays* line A188 |
| 10 | ZmLox3_CDS_A188 - coding sequence of Lox3 from *Zea mays* line A188 |
| 11 | ZmLox3_protein_A188- amino acid sequence of Lox3 from *Zea mays* line A188 |
| 12 | SL57_Zm00001d033623(LOX3)_gDNA - tropical corn line SL57 - genomic sequence of Lox3 from tropical corn line SL57 *(Zea mays)* |
| 13 | SL57_Zm00001d033623(LOX3)_CDS - tropical corn line SL57 - coding sequence of Lox3 from tropical corn line SL57 *(Zea mays)* |
| 14 | SL57_Zm00001d033623(LOX3)_protein - tropical corn line SL57 - amino acid sequence of Lox3 from tropical corn line SL57 *(Zea mays)* |
| 15 | ZmLox3_PH207_genomic - genomic sequence of Lox3 from Zea mays line PH207 |
| 16 | ZmLox3_PH207_CDS - coding sequence of Lox3 from Zea mays line PH207 |
| 17 | ZmLox3_PH207_protein - amino acid sequence of Lox3 from Zea mays line PH207 |
| 18 | Primer for Lox3 qRT-PCR:S3460 - Corn - Primer for expression anlysis of Lox3 in *Zea mays* |
| 19 | Primer for Lox3 qRT-PCR: S3461 - Corn - Primer for expression anlysis of Lox3 in *Zea mays* |

(continued)

| SEQ ID NO. | Description |
|---|---|
| 20 | Primer for Ef-Tu qRT-PCR: S3428 - Primer for expression analysis of the housekeeping gene Elongation factor 1-alpha |
| 21 | Primer for Ef-Tu qRT-PCR: S3429 - Primer for expression analysis of the housekeeping gene Elongation factor 1-alpha |
| 22 | Elongation factor 1-alpha (EF1) gene from A188 - coding sequence of Elongation factor 1-alpha (EF1) gene from *Zea mays* line A188 |
| 23 | Elongation factor 1-alpha (EF1) protein from A188 - amino acid sequence of Elongation factor 1-alpha (EF1) gene from *Zea mays* line A188 |
| 24 | ZmPLT5 PRT - amino acid sequence of PLETHORA transcription factor (PLT) from *Zea mays* |
| 25 | ZmPLT5 CDS - coding sequence of PLETHORA transcription factor (PLT) from *Zea mays* |
| 26 | RBP8 PRT - amino acid sequence of regeneration booster protein |
| 27 | RBP8 CDS - coding sequence of regeneration booster protein |
| 28 | LbCpf1-RR PRT - amino acid sequence of Cpf1 from *Lachnospiraceae bacterium RR* |
| 29 | LbCpf1-RR CDS - coding sequence of Cpf1 from *Lachnospiraceae bacterium RR* |
| 30 | tdTomato PRT - amino acid sequence of fluorescent protein tdTomato |
| 31 | tdTomato CDS - coding sequence of fluorescent protein tdTomato |
| 32 | Protospacer m7GEP336 DNA - DNA sequence of protospacer m7GEP336 |
| 33 | Protospacer m7GEP337 DNA - DNA sequence of protospacer m7GEP337 |
| 34 | Protospacer m7GEP338 DNA - DNA sequence of protospacer m7GEP338 |
| 35 | Protospacer m7GEP339 DNA - DNA sequence of protospacer m7GEP339 |
| 36 | Zm00008a004913_pro.pro - amino acid sequence of Lox3 from *Zea mays* mutant Zm00008a004913 |
| 37 | WVP18-09358-016_pro - amino acid sequence of Lox3 from *Zea mays* mutant WVP18-09358-016 |
| 38 | WVP18-09344-014_pro.pro - amino acid sequence of Lox3 from *Zea mays* mutant WVP18-09344-014 |
| 39 | WVP18-09309-014_339-009_pro.pro - amino acid sequence of Lox3 from *Zea mays* mutant WVP18-09309-014 |
| 40 | WVP18-09307-014_pro.pro - amino acid sequence of Lox3 from *Zea mays* mutant WVP18-09307-014 |
| 41 | Zm00008a004913_CDS - coding sequence of Lox3 from *Zea mays* mutant Zm00008a004913 |
| 42 | WVP18-09358-016_CDS.seq - coding sequence of Lox3 from *Zea mays* mutant WVP18-09358-016 |
| 43 | WVP18-09344-014_CDS.seq - coding sequence of Lox3 from *Zea mays* mutant WVP18-09344-014 |
| 44 | WVP18-09309-014_339-009_CDS.seq - coding sequence of Lox3 from *Zea mays* mutant WVP18-09309-014 |
| 45 | WVP18-09307-014_CDS.seq - coding sequence of Lox3 from *Zea mays* mutant WVP18-09307-014 |
| 46 | crRNA1 - DNA sequencing coding for crRNA1 for targeting the OSR lox3 gene |
| 47 | crRNA2.1 - DNA sequencing coding for crRNA2.1 for targeting the OSR lox3 gene |
| 48 | crRNA2.2 - DNA sequencing coding for crRNA2.2 for targeting the OSR lox3 gene |
| 49 | crRNA3 - DNA sequencing coding for crRNA3 for targeting the OSR lox3 gene |
| 50 | pZFNnptll-LbCpf1-tDT-lox3_TTTV - plasmid sequence of pZFNnptll-LbCpf1 -tDT-lox3_TTTV |
| 51 | cruaxxxxxxf02x - forward primer cruaxxxxxxf02x for analyzing the presence of the transgene |
| 52 | cruaxxxxxxr01x - reverse primer cruaxxxxxxr01x for analyzing the presence of the transgene |

(continued)

| SEQ ID NO. | Description |
|---|---|
| 53 | nptIIxxxf01 - forward primer nptIIxxxf01 for analyzing the presence of the transgene |
| 54 | nptIIxxxr01 - reverse primer nptIIxxxr01 for analyzing the presence of the transgene |
| 55 | tDTxxxf04 - forward primer tDTxxxf04 for analyzing the presence of the transgene |
| 56 | tDTxxxr01 - reverse primer tDTxxxr01 for analyzing the presence of the transgene |
| 57 | CruaxxxMGB - probe CruaxxxMGB for analyzing the presence of the transgene |
| 58 | nptIIxxxMGB - probe nptIIxxxMGB for analyzing the presence of the transgene |
| 59 | tDTxxxMGB - probe tDTxxxMGB for analyzing the presence of the transgene |
| 60 | Fwmfor nuclease - fowrad primer LbCpf1 for nuclease test |
| 61 | LbCpf1- Rv- reverse primer LbCpf1 for nuclease test DNA- LbCpf1 |
| 62 | Probe - probe for nuclease test |
| 63 | IR106_lox3_F1 - primer IR106_lox3_F1 for editing profile analysis |
| 64 | IR106_lox3_F2 - primer IR106_lox3_F2 for editing profile analysis |
| 65 | IR106_lox3_F3 - primer IR106_lox3_F3 for editing profile analysis |
| 66 | IR106_lox3_F4 - primer IR106_lox3_F4 for editing profile analysis |
| 67 | IR106_lox3_R1 - primer IR106_lox3_R1 for editing profile analysis |
| 68 | IR106_lox3_R2 - primer IR106_lox3_R2 for editing profile analysis |
| 69 | IR106_lox3_R3 - primer IR106_lox3_R3 for editing profile analysis |
| 70 | IR106_lox3_R4 - primer IR106_lox3_R4 for editing profile analysis |
| 71 | 946R - primer 946R for amplicon sequencing |
| 72 | 1145F - primer 1145F for amplicon sequencing |
| 73 | 346F - primer 346F for amplicon sequencing |
| 74 | 1948R - primer 1948R for amplicon sequencing |
| 75 | OSR Lox3 genomic DNA BnaC08g37760D_At_ortho!og:AT1G17420 - genomic DNA sequence of Lox3 from oil seed rape *(Brassica napus)* |
| 76 | OSR Lox3 genomic DNA BnaA08g23120D_At_ortholog: AT1G17420 - genomic DNA sequence of Lox3 from oil seed rape *(Brassica napus)* |
| 77 | OSR Lox3 genomic DNA BnaA09g45010D_At_ortholog:AT1G17420 - genomic DNA sequence of Lox3 from oil seed rape *(Brassica napus)* |
| 78 | OSR Lox3 genomic DNA BnaC08g48320D_At_ortholog:AT1G17420 - genomic DNA sequence of Lox3 from oil seed rape *(Brassica napus)* |
| 79 | OSR Lox3 PRT BnaC08g37760D_At_ortholog:AT1G17420 - amino acid sequence of Lox3 from oil seed rape *(Brassica napus)* |
| 80 | OSR Lox3 PRT BnaA08g23120D_At_ortholog:AT1G17420 - amino acid sequence of Lox3 from oil seed rape *(Brassica napus)* |
| 81 | OSR Lox3 PRT BnaA09g45010D_At_ortholog:AT1G17420 - amino acid sequence of Lox3 from oil seed rape *(Brassica napus)* |
| 82 | OSR Lox3 PRT BnaC08g48320D_At_ortholog:AT1G17420 - amino acid sequence of Lox3 from oil seed rape *(Brassica napus)* |
| 83 | OSR Lox3 CDS BnaC08g37760D_At_ortholog: AT1G17420 - coding sequence of Lox3 from oil seed rape *(Brassica napus)* |

(continued)

| SEQ ID NO. | Description |
|---|---|
| 84 | OSR Lox3 CDS BnaA09g45010D_At_ortholog:AT1G17420 - coding sequence of Lox3 from oil seed rape *(Brassica napus)* |
| 85 | OSR Lox3 CDS BnaA09g45010D_At_ortholog:AT1G17420 - coding sequence of Lox3 from oil seed rape *(Brassica napus)* |
| 86 | OSR Lox3 CDS BnaC08g48320D_At_ortholog:AT1G17420 - coding sequence of Lox3 from oil seed rape *(Brassica napus)* |
| 87 | Zm00001eb054040_B73AGP05_T002_CDS - coding sequence of an alternative transcript of Lox3 from *Zea mays* |
| 88 | Zm00001eb054040_B73AGP05_T002_genomic - genomic sequence coding for an alternative transcript of Lox3 from *Zea mays* |
| 89 | Zm00001eb054040_B73AGP05_T002_pro - amino acid sequence of an alternative splice variant of Lox3 from *Zea mays* |

**Definitions**

A plant exhibits "resistance" or "tolerance" to an insect or fungal pathogen, when symptoms of infestation are reduced or not observed at all when the plant is exposed to the pathogen under conditions allowing infestation. Indirectly, resistance and tolerance can also be observed by looking at the performance of the pathogen on resistant or tolerant plants compared to plants with no resistance or tolerance. For example, the amount, size or weight of pathogen can be observed and compared. Larval weight of insects may e.g. be reduced in resistant or tolerant plants compared to a control. Typically, a resistant or tolerant plant shows reduced yield loss compared to a non-resistant plant infested with the same pathogen under the same conditions. Ideally, in a resistant plant, the yield loss caused by infestation is completely compensated, meaning that the plant produces as much yield as a plant, which was not exposed to the pathogen at all but grown under the same conditions.

"Insect pathogens" or "fungal pathogens" are any insects or fungi, which invade and infest crop plants and cause damage and ultimately yield loss in crop plants. For each crop plant, a number of insect or fungal pathogens are known to the skilled person. In some cases, fungal diseases are favoured by the mechanical damage caused by invading insects so that damage can be exaggerated. Therefore, resistance or tolerance to insect pests can also provide a degree of fungal resistance.

The terms "RNA interference" or "RNAi" or "RNA silencing" or "gene silencing" as used herein interchangeably refer to a gene down-regulation (or knock-down) mechanism meanwhile demonstrated to exist in all eukaryotes. The mechanism was first recognized in plants where it was called "post-transcriptional gene silencing" or "PTGS". In RNAi, small RNAs function to guide specific effector proteins to a target nucleotide sequence by complementary base pairing resulting in degradation of the target. A "gene silencing construct" or "RNAi construct" usually comprises so called "sense" and "antisense" sequences. Sense and an antisense sequences are complementary sequences, which are present in reverse orientation in a nucleic acid sequence. If a nucleic acid construct comprises a sense and a corresponding antisense sequence, the two complementary sequences form an RNA double strand upon transcription, which results in an "RNA hairpin". In an RNA hairpin, sense sequences and corresponding antisense sequences, together form a double strand and are separated by an "intervening intron loop sequence" forming the loop of the hairpin structure. An "RNAi construct" may also comprise more than one sense and antisense pair and form several loops.

A "genome editing system" refers to a combination of a site-specific nuclease or site-specific nickase or a functional active fragment or variant thereof together with the cognate guide RNA (or pegRNA or crRNA) guiding the relevant CRISPR nuclease to its target site to be cleaved. A "site-specific nuclease" refers to a nuclease or an active fragment thereof, which is capable to specifically recognize and cleave DNA at a certain target site. Such nucleases typically produce a double strand break (DSB), which is then repaired by nonhomologous end-joining (NHEJ) or homologous recombi-nation (HR). The nucleases include zinc-finger nucleases, transcription activator-like effector nucleases, engineered homing endonucleases, recombinases, transposases and meganucleases and CRISPR nucleases and/or any combination, variant or active fragment thereof. The genome editing system may be a CRISPR/Cas system including CRISPR/Cas9 systems, CRISPR/Cas13 systems, CRISPR/Cpf1 (CRISPR/Cas12a) systems, CRISPR/C2C2 systems, CRISPR/CasX systems, CRISPR/CasY systems, CRISPR/Cmr systems, CRISPR/Csm systems, CRISPR/MAD2 systems, CRISPR/MAD7 systems, CRISPR/CasZ systems, or catalytically active fragment thereof. The "guide molecule", in particular the "guide RNA" (gRNA) may be a trans-activating CRISPR RNA (tracrRNA) plus a synthetic CRISPR RNA (crRNA) or a single guide RNA (sgRNA), which comprises the sequence information targeting the genomic sequence for cleavage by the nuclease.

A "CRISPR nuclease", as used herein, is any nuclease which has been identified in a naturally occurring CRISPR system, which has subsequently been isolated from its natural context, and which preferably has been modified or combined into a recombinant construct of interest to be suitable as tool for targeted genome engineering. Any CRISPR nuclease can be used and optionally reprogrammed or additionally mutated to be suitable for the various embodiments according to the present invention as long as the original wild-type CRISPR nuclease provides for DNA recognition, i.e., binding properties. Said DNA recognition can be PAM (protospacer adjacent motif) dependent. CRISPR nucleases having optimized and engineered PAM recognition patterns can be used and created for a specific application. The expansion of the PAM recognition code can be suitable to target site-specific effector complexes to a target site of interest, independent of the original PAM specificity of the wild-type CRISPR-based nuclease. Cpf1 variants can comprise at least one of a S542R, K548V, N552R, or K607R mutation, preferably mutation S542R/K607R or S542R/K548V/N552R in AsCpf1 from Acidaminococcus. Furthermore, modified Cas or Cpf1 variants or any other modified CRISPR effector variants, e.g., Cas9 variants, can be used according to the methods of the present invention as part of a base editing complex, e.g. BE3, VQR-BE3, EQR-BE3, VRER-BE3, SaBE3, SaKKH-BE3 (see Kim et al., Nat. Biotech., 2017, doi:10.1038/nbt.3803). Therefore, according to the present invention, artificially modified CRISPR nucleases are envisaged, which might indeed not be any "nucleases" in the sense of double-strand cleaving enzymes, but which are nickases or nuclease-dead variants, which still have inherent DNA recognition and thus binding ability. Suitable Cpf1-based effectors for use in the methods of the present invention are derived from Lachnospiraceae bacterium (LbCpf1, e.g., NCBI Reference Sequence: WP_051666128.1), or from Francisella tularensis (FnCpf1, e.g., UniProtKB/Swiss-Prot: A0Q7Q2.1). Variants of Cpf1 are known (cf. Gao et al., BioRxiv, dx.doi.org/10.1101/091611). Variants of AsCpf1 with the mutations S542R/K607R and S542R/K548V/N552R that can cleave target sites with TYCV/CCCC and TATV PAMs, respectively, with enhanced activities in vitro and in vivo are thus envisaged as site-specific effectors according to the present invention. Genome-wide assessment of off-target activity indicated that these variants retain a high level of DNA targeting specificity, which can be further improved by introducing mutations in non-PAM-interacting domains. Together, these variants increase the targeting range of AsCpf1 to one cleavage site for every ~8.7 base pairs (bp) in non-repetitive regions of the human genome, providing a useful addition to the CRISPR/Cas genome engineering toolbox (see Gao et al., supra).

The terms "SDN-1", "SDN-2", and "SDN-3" as used herein are abbreviations for the platform technique "site-directed nuclease" 1, 2, or 3, respectively, as caused by any site directed nuclease of interest, including, for example, Meganucleases, Zinc-Finger Nucleases (ZFNs), Transcription Activator Like Effector Nucleases (TALENs), and CRISPR nucleases. SDN-1 produces a double-stranded or single-stranded break in the genome of a plant without the addition of foreign DNA. For SDN-2 and SDN-3, an exogenous nucleotide template is provided to the cell during the gene editing. For SDN-2, however, no recombinant foreign DNA is inserted into the genome of a target cell, but the endogenous repair process copies, for example, a mutation as present in the template to induce a (point) mutation. In contrast, SDN-3 mechanism use the introduced template during repair of the DNA break so that genetic material is introduced into the genomic material.

A "genome modification" in the context of the present invention refers to any change of a (nucleic acid) sequence that results in at least one difference in the (nucleic acid) sequence distinguishing it from the original sequence. In particular, a modification can be achieved by insertion or addition of one or more nucleotide(s), or substitution or deletion of one or more nucleotide(s) of the original sequence or any combination of these.

A "knock-down" of a gene refers to an experimental technique by which the expression of the gen is reduced. A reduced expression can e.g. be achieved by gene silencing. A "knock-out", on the other hand, leads to an abolished expression, i.e. the gene is not expressed at all. This can e.g. be achieved by replacing or interrupting the sequence of the target gene by genome editing.

A nucleic acid molecule or gene that is "endogenous" to a cell or organism refers to a nucleic acid molecule that naturally occurs in the genome of this cell or organism. On the other hand, a nucleic acid molecule that is "exogenous" to a cell or organism refers to a nucleic acid molecule that does not naturally occur in this cell or organism but has been introduced by a transgenic event.

"Regenerating" a plant, tissue, organ or seed is done by culturing a modified or edited cell in a way that may include steps of de-differentiation and differentiation to obtain specialized tissue or a whole plant, which carries the modification or edit, preferably in every cell. Techniques for regeneration of a plant are well known to the skilled person.

A "nucleic acid construct", "construct" or "expression construct" refers to a nucleic acid molecule encoding or comprising one or more genetic elements, which upon introduction into a target cell can be transcribed and/or translated into a functional form, e.g. RNA(s) or polypeptide(s) or protein(s). A nucleic acid construct may also comprise regulatory sequences such as promoter and terminator sequences facilitating expression of the genetic element(s) as well as spacers and introns. The genetic elements of the present invention can also be encoded on a set of constructs, which constructs can be introduced into a cell simultaneously or consecutively.

A (nucleic acid) construct of the present invention "targets" a genomic sequence or a gene when it contains sequence information, which allows recognition of the genomic sequence orgene and can thus interfere with the sequence, e.g. by site-specific cleavage or silencing.

The targeting can be affected either by direct interaction with the genomic sequence itself or by interaction with the transcript of the genomic sequence. For example, if the nucleic acid construct of the present invention comprises or encodes a sense and a corresponding antisense sequence targeting a genomic sequence, an RNA silencing or RNA interference (RNAi) mechanism is activated upon transcription of the construct, which leads to the destruction of the transcript of the genomic target sequence and thus suppresses expression of the target. In another case, the nucleic acid construct may encode a site-specific nuclease and a guide RNA, which results in cleavage of the target sequence.

"Transformation" or "Transfection" of a plant cell with a construct or set of constructs refers to any established technique to introduce nucleic acid molecules into a cell, such as biolistic approaches (e.g. particle bombardment), microinjection, permeabilising the cell membrane with various treatments such as electroporation or PEG treatment or *Agrobacterium tumefaciens* mediated transformation. Generally, incorporating (a) nucleic acid construct(s), for example by way of transformation, may be accomplished with techniques that are basically known to the person skilled in the art. For example, the nucleic acid construct can be incorporated into the plant cells by infecting a plant tissue or a plant cell with *Agrobacterium tumefaciens* containing the nucleic acid sequence to be transferred in its plasmid that can be integrated into the plant genome. Incorporating by means of a biolistic transfer is another option, wherein the nucleic acid construct to be incorporated into the plant cell is applied to gold particles or tungsten particles, which are then shot into the cells at a high speed. Another option known to the person skilled in the art for incorporating a nucleic acid construct into a plant cell, is the protoplast transformation, wherein either polyethylene glycol is added to the protoplasts in the presence of the nucleic acid molecules to be incorporated, or the protoplasts are exposed to a short current impulse, so that the protoplast membrane transiently becomes permeable for the nucleic acid construct(s).

The term "vector" refers to an element used for introducing a nucleic acid construct or set of nucleic acid constructs into a cellular system. The vector may be a plasmid or plasmid vector, cosmid, artificial yeast artificial chromosomes (YAC), bacterial artificial chromosome (BAC) or P1 artificial chromosomes (PACs), phagemid, bacterial phage based vector, an isolated single-stranded or double-stranded nucleic acid sequence, comprising DNA and RNA sequences in linear or circular form, or a mixture thereof, for introduction or transformation into a plant, plant cell, tissue, organ or material according to the present disclosure.

The terms "plant" or "plant cell" or "part of a plant" as used herein refer to a plant organism, a plant organ, differentiated and undifferentiated plant tissues, plant cells, seeds, and derivatives and progeny thereof. Plant cells include without limitation, for example, cells from seeds, from mature and immature cells or organs, including embryos, meristematic tissues, seedlings, callus tissues in different differentiation states, leaves, flowers, roots, shoots, male or female gametophytes, sporophytes, pollen, pollen tubes and microspores and protoplasts.

"Mutagenesis" refers to a technique, by which modifications or mutations are introduced into a nucleic acid sequence in a random or non- site-specific way. For example, mutations can be induced by certain chemicals such as EMS (ethyl methanesulfonate) or ENU (N-ethyl-N-nitrosourea) or physically, e.g. by irradiation with UV or gamma rays. "Site-specific modifications", on the other hand, rely on the action of site-specific effectors such as nucleases, nickases, recombinases, transposases, base editors. These tools recognize a certain target sequence and allow to introduce a modification at a specific location within the target sequence.

"TILLING" (Targeting Induced Local Lesions in Genomes) is a process, which allows to identify mutations in a specific gene after an (unspecific) mutagenesis has been performed. Mutagenesis may e.g. be performed using a chemical mutagen such as EMS. Then, a sensitive DNA screening technique is used to identify single base mutations. Methods for performing TILLING are known to the skilled person.

Whenever the present disclosure relates to the percentage of identity of nucleic acid or amino acid sequences to each other these values define those values as obtained by using the EMBOSS Water Pairwise Sequence Alignments (nucleotide) programme (www.ebi.ac.uk/Tools/psa/ emboss_water/nucleotide.html) nucleic acids or the EM-BOSS Water Pairwise Sequence Alignments (protein) programme (www.ebi.ac.uk/Toots/psa/emboss_water/) for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see www.ebi.ac.uk/Tools/psa/ and Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a sequence encoding a protein can be "codon-optimized" if the respective sequence is to be used in another organism in comparison to the original organism a molecule originates from.

**Brief Description of Drawings**

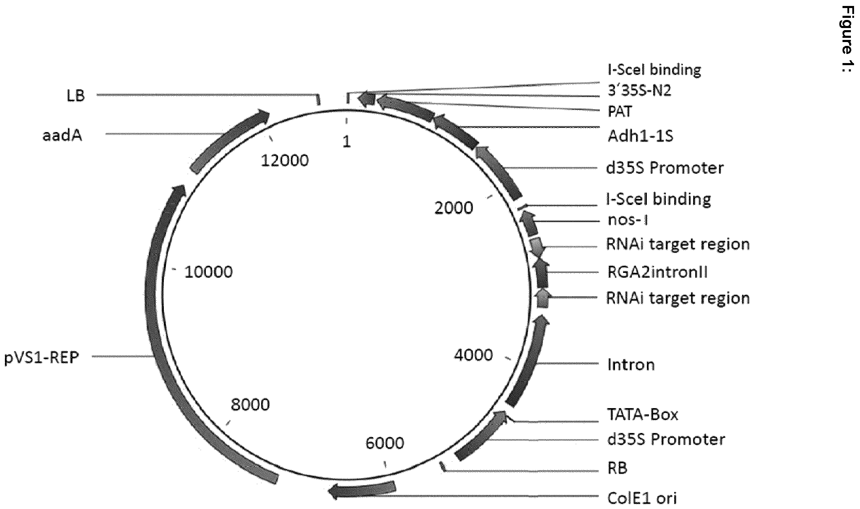

**Figure 1** shows the binary vector used for RNAi mediated downregulation of ZmLox3. An RNAi hairpin construct directed against the ZmLox3 gene was inserted into a binary vector for corn transformation. The T-DNA of the binary vector was transformed into the corn genotype A188. The intron between the d35S promoter and the RNAi target region is the first intron of the maize polyubiquitin gene (ZmUbi; Christensen, A.H., Quail, P.H. Ubiquitin promoter-based vectors for high-level expression of selectable and/or screenable marker genes in monocotyledonous plants. Transgenic Research 5, 213-218 (1996). https://doi.org/10.1007/BF01969712)

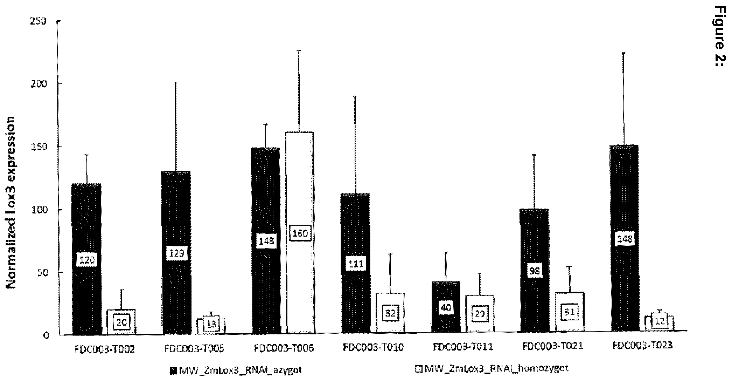

**Figure 2** shows the reduction of ZmLox3 expression in transgenic ZmLox3_RNAi lines. Leaf samples of homozygous GM (genetically modified) plants were analyzed by qRT-PCR for expression of ZmLox3 gene and compared with the gene expression of non-transgenic, azygous plants. The lines FDC003-T002, FDC003-T005, FDC003-T010, FDC003-T021 and FDC003-T023 show a strong reduction of Lox3 expression. The Lox3 expression was normalized against the expression of the house-keeping gene EF1. Mean of 3 and 5 plants are shown. Azygous lines n= 3 plants. GM lines n=5 plants.

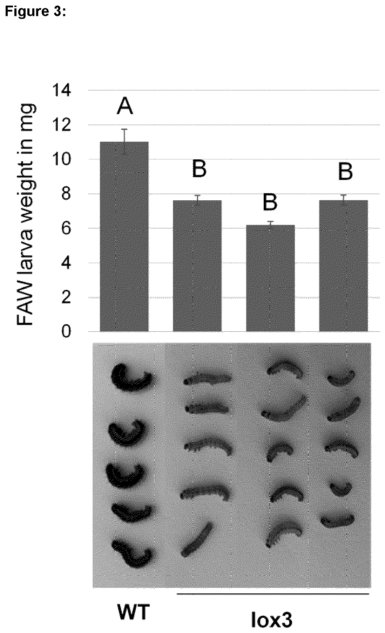

**Figure 3** shows enhanced insect resistance of ZmLox3_RNAi lines. Fall armyworm larva weight (in mg) 10 days after fed on A188 corn leaves (WT (A)) and fed on three different ZmLox3_RNAi lines (B1: FDC003-T002, B2: FDC003-T011 and B3: FDC003-T023). Data are from 3 independent experiments pulled together (mean +/- SE). Different letters represent groups that are significantly different (ANOVA and Tukey's honest significant difference post hoc, n = 75; P < 0.05).

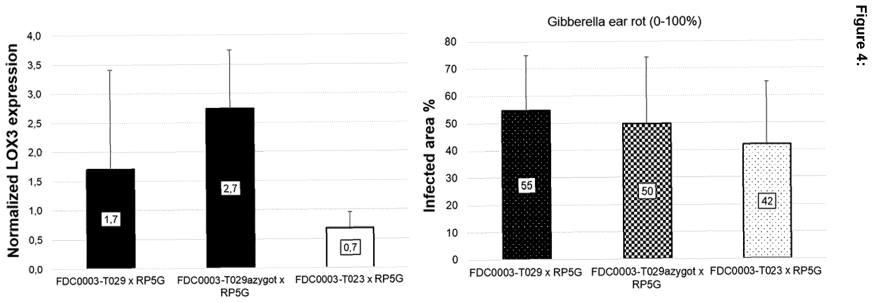

**Figure 4** shows enhanced fungal resistance of ZmLox3_RNAi line FDC0003-T023 Left: Disease scores of *Gibberella* ear rot infected corn cobs of hybrids from FDC0003-T029, FDC0003-T029 azygous and FDC0003-T023 tester line RP5G. Infected area of 40-50 heads of each hybrid was measured 40 days after infection of corn plants with *Fusarium graminearum* in the greenhouse. Right: Lox3 expression of leaves from the hybrids FDC003-T029, FDC0003-T029 azygous and FDC0003-T023 with tester line RP5G at the end of the fungal assay. The Lox3 expression was determined with qRT-PCR and is reduced in the hybrid FDC0003-T023, which shows enhanced fungal resistance and insect resistance.

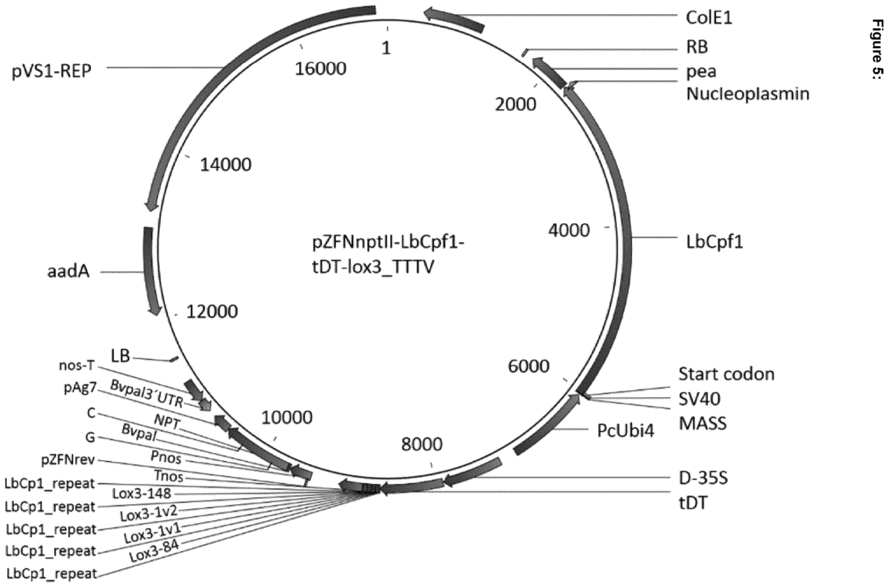

**Figure 5** shows the binary vector used for generation of Lox3 SDN-1 knock-down in tropical corn by genome editing.

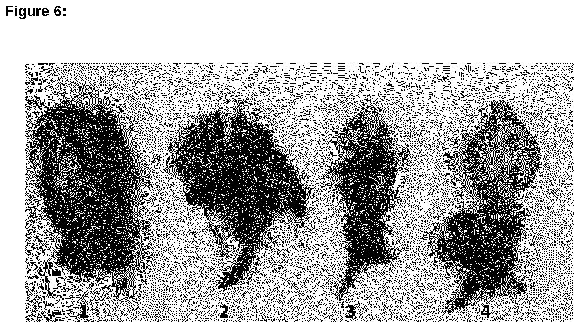

**Figure 6** shows the scoring scheme of the clubroot test performed in Example 11.

**Detailed Description**

The present invention provides means and methods to confer resistance or tolerance to several relevant insect pathogens and, optionally - in addition - fungal pathogens, in plants. In particular, highly damaging diseases in corn (Zm) and oil seed rape (OSR) are addressed in the following disclosure. In some cases, fungal diseases are favoured by the mechanical damage caused by invading insects so that damage can be exaggerated. It was thus shown, that resistance or tolerance to insect pests can also provide a degree of fungal resistance or tolerance. Insect infestation in crop plants is often managed using insecticide treatments and Bt traits, i.e. genetically modified (GM) plants, which produce insecticidal toxins. The present invention provides a novel approach to manage insect pests using a non-GM trait. Through a product development by TILLING, a non-GM trait increasing insect tolerance could be achieved. This can be used in addition to pesticides to reduce their use and/or to protect the crop against insects when pesticides cannot be applied (e.g. when it rains). It is also envisioned to use the approach described herein as a stacked trait with GM trait (Bt crops) to slow down the breakdown of insect resistance to GM crops. Additionally, a knock-down using gene silencing and a knock-out using genome editing techniques could be achieved.

In one preferred aspect, the present invention relates to a method for conferring or increasing resistance or tolerance to fall armyworm (Spodoptera frugiperda) and, optionally to a fungal pathogen, in particular Fusarium species, to/in a plant, preferably a maize (Zea mays) plant, comprising the steps of:

(i) providing at least one plant cell, preferably a maize plant cell;

(ii) introducing into the at least one plant cell at least one gene silencing construct, at least one genome editing system or a genome modification, which leads to a targeted knock-down or a knock-out of a Lox3 gene endogenous to the plant;

(iii) obtaining at least one modified plant cell having reduced or abolished expression of the Lox3 gene; and

(iv) obtaining at least one plant cell, tissue, organ, plant or seed having reduced or abolished expression of the Lox3 gene, optionally after an additional step of regenerating the plant tissue, organ, plant or seed from the at least one modified cell.

In another aspect, the present invention relates to a method for conferring or increasing resistance or tolerance to an insect and, optionally a fungal pathogen to/in a plant comprising the steps of:

(i) providing at least one plant cell;

(ii) introducing into the at least one plant cell at least one gene silencing construct, at least one genome editing system or a genome modification, which leads to a targeted knock-down or a knock-out of a Lox3 gene endogenous to the plant;

(iii) obtaining at least one modified plant cell having reduced or abolished expression of the Lox3 gene; and

(iv) obtaining at least one plant cell, tissue, organ, plant or seed having reduced or abolished expression of the Lox3 gene, optionally after an additional step of regenerating the plant tissue, organ, plant or seed from the at least one modified cell.

It was established in the context of the present invention that reduced or abolished expression of the endogenous Lox3 gene in plants confers or increases resistance to insect pests and can at the same time also provide a degree of resistance to fungal pathogens. To reduce or abolish expression of the endogenous Lox3 gene, three different approaches may be taken: Lox3 expression may be reduced by gene silencing using an RNAi hairpin construct, which contains as sense and antisense sequences sections of the endogenous Lox3 gene. Alternatively, a genome editing system can be used, which introduces a double strand break (DSB) at or in the endogenous Lox3 sequence resulting in a disruption or (partial) replacement of the Lox3 locus. Finally, it is also possible to introduce mutations into the endogenous Lox3 by random mutagenesis and identify such mutations, which provide the desired knock-down or knock-out effect.

In a preferred embodiment, the method described above is for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species*, *Colletotrichum species*, in particular *Colletotrichum graminicola* and *Colletotrichum sublineolum*, *Diplodia species*, *Cercospora zeina* and *Cercospora zeae-maydis* to/in maize *(Zea mays)*.

The above mentioned pathogens cause a lot of damage to corn and reduce yields significantly. Using the approaches described herein, resistance or tolerance to these pathogens can be conferred or increased. Another important crop plant, oil seed rape, may also suffer from certain insect or fungal pests.

In another preferred embodiment, the method described above therefore is for conferring or increasing resistance or tolerance to one or more insect(s) selected from the group consisting of green peach aphid *(Myzus persicae),* diamondback moth *(Plutella xylostella),* cabbage stem flea beetle *(Psylliodes chrysocephala),* crucifer flea beetle *(Phyllotreta cruciferae),* striped flea beetle *(Phyllotreta striolata),* hop flea beetle *(Psylliodes punctulata),* rape stem weevil *(Ceutorhynchus picitarsis)* and cabbage stem weevil *(Ceutorhynchus quadridens)* and, optionally conferring or increasing resistance or tolerance to one or more fungal pathogen(s) selected from the group consisting of *Phoma lingam* and *Plasmodiophora brassicae* to/in oilseed rape *(Brassica napus)*.

The Lox3 gene in corn *(Zea mays)* is represented by the genomic DNA sequence of SEQ ID NO: 6, while the coding sequence is represented by the sequence of SEQ ID NO: 7. For the corn lines A188, a tropical corn line, the line PH207 and an alternative transcript, the genomic sequences and coding sequences are respectively represented by the sequences of SEQ ID NOs: 9 (A188, genomic DNA), 10 (A188, coding sequence), 12 (tropical corn line, genomic DNA), 13 (tropical corn line, coding sequence), 15 (PH207, genomic DNA), 16 (PH207, coding sequence), 87 (alternative transcript, coding sequence) and 88 (alternative transcript, genomic sequence).

In one embodiment of the method described above, the Lox3 gene is therefore represented by a nucleic acid sequence of SEQ ID NO: 6, 7, 9, 10, 12, 13, 15, 16, 87 or 88 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 6, 7, 9, 10, 12, 13, 15, 16, 87 or 88.

The protein (amino acid) sequences of Lox3 in corn *(Zea mays)* translated from the above nucleic acid sequences are represented by the sequences of SEQ ID NOs: 8 (ZmLox3 protein), 11 (A188, ZmLox3 protein), 14 (tropical corn line, ZmLox3 protein), 17 (PH207, ZmLox3 protein) and 89 (alternative transcript, ZmLox3 protein).

In one embodiment of the method described above, the Lox3 gene therefore encodes an amino acid sequence of SEQ ID NO: 8, 11, 14, 17 or 89 an amino acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 8, 11, 14, 17 or 89.

The Lox3 gene in oil seed rape *(Brassica napus)* is represented by the genomic DNA sequences of SEQ ID NOs: 75, 76, 77 and 78 while the coding sequences are represented by the sequences of SEQ ID NOs: 83, 84, 85 and 86.

In one embodiment of the method described above, the Lox3 gene is therefore represented by a nucleic acid sequence of SEQ ID NO: 75, 76, 77, 78, 83, 84, 85 or 86 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: : 75, 76, 77, 78, 83, 84, 85 or 86.

The protein (amino acid) sequences of Lox3 in oil seed rape *(Brassica napus)* translated from the above nucleic acid sequences are represented by the sequences of SEQ ID NOs: 79, 80, 81 and 82.

In one embodiment, the Lox3 gene therefore encodes an amino acid sequence of SEQ ID NO: 79, 80, 81 or 82 or an amino acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 79, 80, 81 or 82.

In a further embodiment of the method according to any of the embodiments described above, in step (ii) a construct is introduced into the at least one plant cell, which targets the Lox3 gene for gene silencing.

RNAi techniques for targeted gene silencing are well known in the art. To this end, an RNAi construct is introduced into a plant cell, which contains sequence information of the genomic target to be silenced in the cell. The construct is preferably introduced in form of a DNA sequence, which is then transcribed into functional RNA in the cell. In particular, the RNAi construct encodes sense and antisense sequences, which represents (a fragment of) the genomic target. The complementary sense and antisense sequences, which are present in reverse orientation in the construct form an RNA double strand upon transcription, which results in an RNA hairpin with an intervening intron loop sequence. The presence of the RNAi construct ultimately results in a reduced expression of the target, i.e. a knock-down.

In one embodiment of the method described above, the construct encodes an RNAi construct comprising a sense and an antisense sequence targeting the Lox3 gene, the RNAi construct forming an RNA hairpin upon transcription.

In a preferred embodiment of the method described above, in step (ii) a construct is introduced into the at least one plant cell, which targets the Lox3 gene for gene silencing wherein the construct is or wherein the construct encodes an RNAi construct comprising a sense and an antisense sequence targeting the Lox3 gene, the RNAi construct forming an RNA hairpin upon transcription,

(a) wherein the sense sequence is encoded by a nucleic acid sequence of SEQ ID NO: 1, or a nucleic acid sequence having a sequence identity of at least 90%, 91%, 92%, 93%, 94%, 95%, 96%, 97%, 98% or at least 99% to the sequence of SEQ ID NO: 1; and/or

(b) wherein the antisense sequence is encoded by a nucleic acid sequence of SEQ ID NO: 2, or a nucleic acid sequence having a sequence identity of at least 90%, 91%, 92%, 93%, 94%, 95%, 96%, 97%, 98% or at least 99% to the sequence of SEQ ID NO: 2, and/or

(c) preferably wherein the construct comprises a nucleic acid sequence of SEQ ID NO: 4, or a nucleic acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4, optionally wherein the construct is introduced into the at least one plant cell by transformation or transfection mediated by biolistic bombardment, Agrobacterium-mediated transformation, micro- or nanoparticle delivery, chemical transfection, or a combination thereof.

Preferably, the sense and antisense sequences have a length of 40 to 500 nucleotides, more preferably 100 to 300 nucleotides.

In one embodiment of the method described above, the RNA hairpin has an intervening intron loop sequence comprising a nucleic acid sequence of SEQ ID NO: 3, or a nucleic acid sequence having a sequence identity of at least 85%, 86%, 87%, 88%, 89%, 90%, 91%, 92%, 93%, 94%, 95%, 96%, 97%, 98% or at least 99% to the sequence of SEQ ID NO: 3.

In another embodiment of the method described above, in step (ii) a construct is introduced into the at least one plant cell, which targets the Lox3 gene for gene silencing, preferably wherein the construct is introduced as part of a vector the vector being introduced into the plant cell, which vector comprises or consists of a nucleic acid sequence of SEQ ID NO: 5, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 5.

In one embodiment of the method described in any of the embodiment described above, the method is for conferring or increasing resistance or tolerance to one or more insect(s) selected from the group consisting of fall army worm *(Spodoptera frugiperda)*, corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays)* and the sense sequence is encoded by a nucleic acid sequence of SEQ ID NO: 1, or a nucleic acid sequence having a sequence identity of at least 90%, 91%, 92%, 93%, 94%, 95%, 96%, 97%, 98% or at least 99% to the sequence of SEQ ID NO: 1.

In another embodiment of the method described in any of the embodiment described above, the method is for conferring or increasing resistance or tolerance to one or more insect(s) selected from the group consisting of fall army worm *(Spodoptera frugiperda)*, corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays)* and the antisense sequence is encoded by a nucleic acid sequence of SEQ ID NO: 2, or a nucleic acid sequence having a sequence identity of at least 90%, 91%, 92%, 93%, 94%, 95%, 96%, 97%, 98% or at least 99% to the sequence of SEQ ID NO: 2.

In yet another embodiment of the method described in any of the embodiment described above, the method is for conferring or increasing resistance or tolerance to one or more insect(s) selected from the group consisting of fall army worm *(Spodoptera frugiperda)*, corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays)* and the RNA hairpin has an intervening intron loop sequence comprising a nucleic acid sequence of SEQ ID NO: 3, or a nucleic acid sequence having a sequence identity of at least 85%, 86%, 87%, 88%, 89%, 90%, 91%, 92%, 93%, 94%, 95%, 96%, 97%, 98% or at least 99% to the sequence of SEQ ID NO: 3.

In one embodiment of the method described in any of the embodiment described above, the method is for conferring or increasing resistance or tolerance to one or more insect(s) selected from the group consisting of fall army worm *(Spodoptera frugiperda)*, corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays)* and the construct comprises a nucleic acid sequence of SEQ ID NO: 4, or a nucleic acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4.

The RNAi construct may preferably be introduced on a vector encoding the RNAi hairpin, which is formed upon transcription.

In one embodiment of the method according to any of the embodiments described above, the method is for conferring or increasing resistance or tolerance to one or more insect(s) selected from the group consisting of fall army worm *(Spodoptera frugiperda)*, corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays)* and a vector is introduced into the plant cell, which vector comprises or consists of a nucleic acid sequence of SEQ ID NO: 5, or a nucleic acid sequence having a sequence identity of at least 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 5.

The introduction of the construct into the plant cell may e.g. be achieved by means of transformation or transfection. Besides transformation methods based on biological approaches, like *Agrobacterium* transformation or viral vector mediated plant transformation, methods based on physical delivery methods, like particle bombardment or microinjection, have evolved as prominent techniques for importing genetic material into a plant cell or tissue of interest. Helenius et al. ("Gene delivery into intact plants using the HeliosTM Gene Gun", Plant Molecular Biology Reporter, 2000, 18 (3):287-288) discloses a particle bombardment as physical method for transferring material into a plant cell. Currently, there are a variety of plant transformation methods to introduce genetic material in the form of a genetic construct into a plant cell of interest, comprising biological and physical means known to the skilled person on the field of plant biotechnology, which can be applied. A common biological means is transformation with *Agrobacterium spp.* which has been used for decades for a variety of different plant materials. Viral vector mediated plant transformation represents a further strategy for introducing genetic material into a cell of interest. Physical means finding application in plant biology are particle bombardment, also named biolistic transfection or microparticle-mediated gene transfer, which refers to a physical delivery method for transferring a coated microparticle or nanoparticle comprising a nucleic acid or a genetic construct of interest into a target cell or tissue. Physical introduction means are suitable to introduce nucleic acids, i.e., RNA and/or DNA, and proteins. Likewise, transformation or transfection methods exist for specifically introducing a nucleic acid or an amino acid construct of interest into a plant cell, including electroporation, microinjection, nanoparticles, and cell-penetrating peptides (CPPs). Furthermore, chemical-based transfection methods exist to introduce genetic constructs and/or nucleic acids and/or proteins, comprising *inter alia* transfection with calcium phosphate, transfection using liposomes, e.g., cationic liposomes, or transfection with cationic polymers, including DEAD-dextran or polyethylenimine, or combinations thereof. Every delivery method has to be specifically fine-tuned and optimized so that a construct of interest can be introduced into a specific compartment of a target cell of interest in a fully functional and active way.

In one embodiment of the method described above, the construct is introduced into the at least one plant cell by transformation or transfection mediated by biolistic bombardment, *Agrobacterium-mediated* transformation, micro- or nanoparticle delivery, chemical transfection, or a combination thereof.

As already mentioned above, as an alternative to gene silencing, the knock-down or knock-out of the endogenous Lox3 gene to confer or enhance resistance or tolerance can also be effected by genome editing.

In one embodiment of the method described above, in step (ii) at least one genome editing system is introduced into the at least one cell, which targets the Lox3 gene, wherein the at least one genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same.

Genome editing techniques allow to introduce a double strand break at one or more predetermined target site(s), i.e. by or within the endogenous Lox3 thereby disrupting the Lox3 locus and optionally inserting an exogenous sequence or replacing an endogenous sequence. The double strand break is introduced by a site-specific nuclease such as meganucleases, zinc finger nucleases (ZFNs), transcription activator-like effector nucleases (TALENs), or the clustered regularly interspaced short palindromic repeat (CRISPR) nucleases. The nucleases cause double strand breaks (DSBs) at specific cleaving sites, which are repaired by nonhomologous end-joining (NHEJ) or homologous recombination (HR). The use of a repair template guides the cellular repair process so that the results of the repair are error-free and predictable. A repair template preferably comprises symmetric or asymmetric homology arms, which are complementary to the sequences flanking a double strand break and therefore allow to insert a sequence or close the break in a controlled manner.

In one embodiment of the method described above, the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or the at least one genome editing system is selected from a zinc finger nuclease system, or a transcription activator-like nuclease system, or a meganuclease system, or any combination, variant, or an active fragment thereof.

A preferred Cpf1 (Cas12a) nuclease to be used in the method of the present invention is encoded by the nucleic acid sequence of SEQ ID NO: 29, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 29.

The preferred Cpf1 (Cas12a) nuclease is further represented by the amino acid sequence of SEQ ID NO: 28 or an amino acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 28.

The genome editing system can be introduced by transformation or transfection mediated by biolistic bombardment, *Agrobacterium-mediated* transformation, micro- or nanoparticle delivery, chemical transfection, or a combination thereof as explained in more detail above in the context of the RNAi construct.

A CRISPR system includes the use of a guide RNA (gRNA) or CRISPR (crRNA), which guides the CRISPR nuclease to the target site by sequence recognition.

In a preferred embodiment of the method for conferring or increasing resistance or tolerance to fall armyworm *(Spodoptera frugiperda)* and, optionally to a fungal pathogen, in particular *Fusarium species,* to/in a plant, preferably a maize *(Zea mays)* plant, as described in any of the embodiments above, the at least one genome editing system comprises a protospacer having a nucleic acid sequence of SEQ ID NO: 32, 33, 34 or 35, or a nucleic acid sequence having 85%, 86%, 87%, 88%, 89%, 90%, 91%, 92%, 93%, 94%, 95%, 96%, 97%, 98% or at least 99% to the sequence of any of SEQ ID NOs: 32, 33, 34 or 35.

In one embodiment of the method described in any of the embodiments above, the method is for conferring or increasing resistance or tolerance to one or more insect(s) selected from the group consisting of fall army worm *(Spodoptera frugiperda)*, corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays),* in particular a tropical maize line, and the at least one genome editing system comprises a protospacer having a nucleic acid sequence of SEQ ID NO: 32, 33, 34 or 35, or a nucleic acid sequence having 85%, 86%, 87%, 88%, 89%, 90%, 91%, 92%, 93%, 94%, 95%, 96%, 97%, 98% or at least 99% to the sequence of any of SEQ ID NOs: 32, 33, 34 or 35.

In one embodiment of the method described in any of the embodiments above, the method is for *conferring* or increasing resistance or tolerance to one or more insect(s) selected from the group consisting of green peach aphid *(Myzus persicae),* diamondback moth *(Plutella xylostella),* cabbage stem flea beetle *(Psylliodes chrysocephala),* crucifer flea beetle *(Phyllotreta cruciferae),* striped flea beetle *(Phyllotreta striolata),* hop flea beetle *(Psylliodes punctulata),* rape stem weevil *(Ceutorhynchus picitarsis)* and cabbage stem weevil *(Ceutorhynchus quadridens) and, optionally* conferring or increasing resistance or tolerance to one or more fungal pathogen(s) selected from the group consisting of *Phoma lingam* and *Plasmodiophora brassicae to/in oilseed rape (Brassica napus), and* the at least one genome editing system comprises a crRNA encoded by a nucleic acid sequence of any of SEQ ID NOs: 46 to 49, or a nucleic acid sequence having a sequence identity of at least 70%, 71%, 72%, 73%, 74%, 75%, 76%, 77%, 78%, 79%, 80%, 81 %, 82%, 83%, 84%, 85%, 86%, 87%, 88%, 89%, 90%, 91%, 92%, 93%, 94%, 95%, 96%, 97%, 98% or at least 99% to the sequence of any of SEQ ID NOs: 46 to 49.

In one embodiment of this method, the genome editing system is encoded by a plasmid of the nucleic acid sequence of SEQ ID NO: 50 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 50.

A further alternative to introduce a modification, which results in reduced or abolished expression of endogenous Lox3, is by mutagenesis. From a range of mutants, the ones with the desired knock-down or knock-out can then be identified, e.g. by TILLING.

In one embodiment of the method described above, in step (ii) a mutagenesis is performed on a single or on a plurality of cell(s) by applying chemicals or radiation.

Preferably, an alkylating agent, in particular ethyl methanesulfonate is applied to the single or the plurality of cell(s) to induce mutagenesis.

In one embodiment of the method described above, one or more mutations in the Lox3 gene are inserted and identified by TILLING in step (ii).

In another embodiment of the method described above, one or more cell(s) with knock-down or knock-out mutations in the Lox3 gene are selected in step (ii).

In one embodiment of the method described above, the maize mutant selected in step (ii) comprises a Lox3 with an amino acid sequence selected from the sequences of SEQ ID NOs: 36 to 40 or an amino acid sequence having a sequence identity of at least 70%, 71 %, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of any of SEQ ID NOs: 36 to 40.

In another embodiment of the method described above, the maize mutant selected in step (ii) comprises a Lox3 encoded by a nucleic acid sequence selected from the sequences of SEQ ID NOs: 41 to 45 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of any of SEQ ID NOs: 41 to 45.

In a preferred embodiment of the method for conferring or increasing resistance or tolerance to fall armyworm *(Spodoptera frugiperda)* and, optionally to a fungal pathogen, in particular *Fusarium species,* to/in a plant, preferably a maize *(Zea mays)* plant, described in any of the embodiments above, in step (ii) a mutagenesis is performed and maize mutant is selected, which comprises a Lox3 with an amino acid sequence selected from the sequences of SEQ ID NOs: 36 to 40 or an amino acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of any of SEQ ID NOs: 36 to 40.

In another preferred embodiment of the method for conferring or increasing resistance or tolerance to fall armyworm *(Spodoptera frugiperda)* and, optionally to a fungal pathogen, in particular *Fusarium species,* to/in a plant, preferably a maize *(Zea mays)* plant, described in any of the embodiments above, in step (ii) a mutagenesis is performed and maize mutant is selected, which comprises a Lox3 encoded by a nucleic acid sequence selected from the sequences of SEQ ID NOs: 41 to 45 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of any of SEQ ID NOs: 41 to 45.

In another aspect, the present invention relates to a maize cell, maize tissue, maize organ, maize plant or maize seed obtained or obtainable by a method according to any of the embodiments described above.

In a further aspect, the present invention relates to an oilseed rape cell, oilseed rape tissue, oilseed rape organ, oilseed rape plant or oilseed rape seed obtained or obtainable by a method according to any of the embodiments described above.

The present invention also provides expression constructs for specifically targeting the Lox3 gene in maize and in oil seed rape for gene silencing. To obtain such a construct, sequences from the respective endogenous Lox3 gene are used as sense and antisense sequences, which form an RNA hairpin upon transcription in the cell.

In one aspect, the present invention relates to an expression construct, which targets the Lox3 gene in maize for gene silencing, wherein the construct encodes an RNAi construct comprising a sense and an antisense sequence targeting the Lox3 gene endogenous to a maize plant, which RNAi construct forms an RNA hairpin upon transcription.

In another aspect, the present invention relates to an expression construct, which targets the Lox3 gene in oilseed rape for gene silencing, wherein the construct encodes an RNAi construct comprising a sense and an antisense sequence targeting the Lox3 gene endogenous to an oilseed rape plant, which RNAi construct forms an RNA hairpin upon transcription.

Preferably, the sense and antisense sequences have a length of 40 to 500 nucleotides, more preferably 100 to 300 nucleotides.

In a preferred embodiment of the expression construct, the expression construct targets the Lox3 gene in maize for gene silencing and the construct encodes an RNAi construct comprising a sense and an antisense sequence targeting the Lox3 gene endogenous to a maize plant, which RNAi construct forms an RNA hairpin upon transcription, wherein

(a) the sense sequence is encoded by a nucleic acid sequence of SEQ ID NO: 1, or a nucleic acid sequence having a sequence identity of at least 90%, 91%, 92%, 93%, 94%, 95%, 96%, 97%, 98% or at least 99% to the sequence of SEQ ID NO: 1; and/or

(b) wherein the antisense sequence is encoded by a nucleic acid sequence of SEQ ID NO: 2, or a nucleic acid sequence having a sequence identity of at least 90%, 91%, 92%, 93%, 94%, 95%, 96%, 97%, 98% or at least 99% to the sequence of SEQ ID NO: 2, and/or optionally wherein the RNA hairpin has an intervening intron loop sequence comprising a nucleic acid sequence of SEQ ID NO: 3, or a nucleic acid sequence having a sequence identity of at least 85%, 86%, 87%, 88%, 89%, 90%, 91%, 92%, 93%, 94%, 95%, 96%, 97%, 98% or at least 95% to the sequence of SEQ ID NO: 3; and/or

(c) preferably wherein the construct comprises a nucleic acid sequence of SEQ ID NO: 4, or a nucleic acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4.

In one embodiment of the expression construct described above, the sense sequence is encoded by a nucleic acid sequence of SEQ ID NO: 1, or a nucleic acid sequence having a sequence identity of at least 90%, 91%, 92%, 93%, 94%, 95%, 96%, 97%, 98% or at least 99% to the sequence of SEQ ID NO: 1.

In another embodiment of the expression construct described above, the antisense sequence is encoded by a nucleic acid sequence of SEQ ID NO: 2, or a nucleic acid sequence having a sequence identity of at least 90%, 91%, 92%, 93%, 94%, 95%, 96%, 97%, 98% or at least 99% to the sequence of SEQ ID NO: 2.

In yet another embodiment of the expression construct described above, the RNA hairpin has an intervening intron loop sequence comprising a nucleic acid sequence of SEQ ID NO: 3, or a nucleic acid sequence having a sequence identity of at least 85%, 86%, 87%, 88%, 89%, 90%, 91%, 92%, 93%, 94%, 95%, 96%, 97%, 98% or at least 99% to the sequence of SEQ ID NO: 3.

In a further embodiment of the expression construct described above, the construct comprises a nucleic acid sequence of SEQ ID NO: 4, or a nucleic acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4.

In another aspect, the present invention relates to a vector comprising or consisting of a nucleic acid sequence of SEQ ID NO: 5 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 5.

In another aspect, the present invention relates to an RNAi hairpin construct for conferring or increasing resistance or tolerance to fall armyworm *(Spodoptera frugiperda)* and, optionally to a fungal pathogen, in particular *Fusarium species,* to/in a maize plant *(Zea mays),* wherein the RNAi hairpin construct comprises a nucleic acid sequence of SEQ ID NO: 4, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4.

In yet another aspect, the present invention relates to an RNAi hairpin construct conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays),* wherein the RNAi hairpin construct comprises a nucleic acid sequence of SEQ ID NO: 4, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4.

The present invention also provides constructs, which target the endogenous Lox3 gene in maize and oilseed rape for genome editing. Such genome editing systems comprise a site-specific nuclease, in case of a CRISPR based system, a guide RNA and, optionally a repair template.

In one aspect the present invention relates to an expression construct encoding a genome editing system, which targets the Lox3 gene in maize, wherein the genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same.

In a preferred embodiment of the construct described above, preferably the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or the at least one genome editing system is selected from a zinc finger nuclease system, or a transcription activator-like nuclease system, or a meganuclease system, or any combination, variant, or an active fragment thereof.

In one embodiment of the expression construct described in any of the embodiments above, the expression construct comprises a protospacer having a nucleic acid sequence of SEQ ID NO: 32, 33, 34 or 35, or a nucleic acid sequence having 85%, 86%, 87%, 88%, 89%, 90%, 91%, 92%, 93%, 94%, 95%, 96%, 97%, 98% or at least 99% to the sequence of any of SEQ ID NOs: 32, 33, 34 or 35.

In another aspect, the present invention relates to an expression construct encoding a genome editing system, which targets the Lox3 gene in oilseed rape, wherein the genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same.

In one embodiment of the expression construct described above, the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or the at least one genome editing system is selected from a zinc finger nuclease system, or a transcription activator-like nuclease system, or a meganuclease system, or any combination, variant, or an active fragment thereof.

In one embodiment of the expression construct described above, the expression construct comprises a crRNA encoded by a nucleic acid sequence of any of SEQ ID NOs: 46 to 49, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of any of SEQ ID NO: 46 to 49.

In another embodiment of the expression construct described above, the genome editing system is encoded by a plasmid of the nucleic acid sequence of SEQ ID NO: 50 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 50.

In one embodiment of the expression construct described above, the expression construct comprises a protospacer having a nucleic acid sequence of SEQ ID NO: 32, 33, 34 or 35, or a nucleic acid sequence having 85%, 86%, 87%, 88%, 89%, 90%, 91%, 92%, 93%, 94%, 95%, 96%, 97%, 98% or at least 99% to the sequence of any of SEQ ID NOs: 32, 33, 34 or 35.

In yet another aspect, the present invention relates to a vector encoding an expression construct according to any of the embodiments described above.

The present invention also provides a maize cell, maize tissue, maize organ, maize plant or maize seed or an oilseed rape cell, oilseed rape tissue, oilseed rape organ, oilseed rape plant or oilseed rape seed comprising an expression construct or a vector according to any of the embodiments described above.

Furthermore, the present invention provides a use of an expression construct according to any of the embodiments described above for conferring or increasing resistance or tolerance to an insect and, optionally a fungal pathogen to/in a plant, in particular for use in a method according to any of the embodiments described above.

In a preferred embodiment of the use described above, at least one gene silencing construct and/or of at least one genome editing system and/or of at least one genome modification as defined in any of the embodiments described above, which leads to a targeted knock-down or a knock-out of the endogenous a Lox3 gene is used for conferring or increasing resistance or tolerance to fall armyworm *(Spodoptera frugiperda)* and, optionally to a fungal pathogen, in particular *Fusarium species,* to/in a plant, preferably a maize *(Zea mays)* plant.

In one aspect, the present invention relates to a use of at least one gene silencing construct, at least one genome editing system or a genome modification, which leads to a targeted knock-down or a knock-out of the endogenous a Lox3 gene, for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays).*

In another aspect, the present invention relates to a use of at least one gene silencing construct, at least one genome editing system or a genome modification, which leads to a targeted knock-down or a knock-out of the endogenous a Lox3 gene, for conferring or increasing resistance or tolerance to one or more insect(s) selected from the group consisting of green peach aphid *(Myzus persicae),* diamondback moth *(Plutella xylostella),* cabbage stem flea beetle *(Psylliodes chrysocephala),* crucifer flea beetle *(Phyllotreta cruciferae),* striped flea beetle *(Phyllotreta striolata),* hop flea beetle *(Psylliodes punctulata),* rape stem weevil *(Ceutorhynchus picitarsis)* and cabbage stem weevil *(Ceutorhynchus quadridens)* and, optionally conferring or increasing resistance or tolerance to one or more fungal pathogen(s) selected from the group consisting of *Phoma lingam* and *Plasmodiophora brassicae* to/in oilseed rape *(Brassica napus).*

According to one approach described in detail above, an RNAi or gene silencing construct may be used for conferring or increasing resistance or tolerance to an insect and, optionally a fungal pathogen to/in a plant.

In one aspect, the present invention therefore relates to the use of a construct, the construct being or encoding an RNAi construct comprising a sense and an antisense sequence targeting the endogenous Lox3 gene of a maize plant, wherein the RNAi construct forms an RNA hairpin upon transcription, for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays).*

In another aspect, the present invention relates to the use of a construct, the construct being or encoding an RNAi construct comprising a sense and an antisense sequence targeting the endogenous Lox3 gene of an oilseed rape plant, wherein the RNAi construct forms an RNA hairpin upon transcription, for conferring or increasing resistance or tolerance to one or more insect(s) selected from the group consisting of green peach aphid *(Myzus persicae),* diamondback moth *(Plutella xylostella),* cabbage stem flea beetle *(Psylliodes chrysocephala),* crucifer flea beetle *(Phyllotreta cruciferae),* striped flea beetle *(Phyllotreta striolata),* hop flea beetle *(Psylliodes punctulata),* rape stem weevil *(Ceutorhynchus picitarsis)* and cabbage stem weevil *(Ceutorhynchus quadridens)* and, optionally conferring or increasing resistance or tolerance to one or more fungal pathogen(s) selected from the group consisting of *Phoma lingam* and *Plasmodiophora brassicae* to/in oilseed rape *(Brassica napus).*

In one embodiment of the use described above, the sense sequence is encoded by a nucleic acid sequence of SEQ ID NO: 1, or a nucleic acid sequence having a sequence identity of at least 90%, 91%, 92%, 93%, 94%, 95%, 96%, 97%, 98% or at least 99% to the sequence of SEQ ID NO: 1.

In another embodiment of the use according to any of the embodiments described above, the antisense sequence is encoded by a nucleic acid sequence of SEQ ID NO: 2, or a nucleic acid sequence having a sequence identity of at least 90%, 91%, 92%, 93%, 94%, 95%, 96%, 97%, 98% or at least 99% to the sequence of SEQ ID NO: 2.

In yet another embodiment of the use according to any of the embodiments described above, the RNA hairpin has an intervening intron loop sequence comprising a nucleic acid sequence of SEQ ID NO: 3, or a nucleic acid sequence having a sequence identity of at least 85%, 86%, 87%, 88%, 89%, 90%, 91%, 92%, 93%, 94%, 95%, 96%, 97%, 98%, or at least 99% to the sequence of SEQ ID NO: 3.

In one embodiment of the use described above, the construct comprises a nucleic acid sequence of SEQ ID NO: 4, or a nucleic acid sequence having a sequence identity of at least 80%, 81%, 82%, 83%, 84%, 85%, 86%, 87%, 88%, 89%, 90%, 91 %, 92%, 93%, 94%, 95%, 96%, 97%, 98% or at least 99% to the sequence of SEQ ID NO: 4.

The present invention also relates to the use of a vector, which vector comprises or consists of a nucleic acid sequence of SEQ ID NO: 5, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 5 for conferring or increasing resistance or tolerance to one or more insect(s) selected from the group consisting of fall army worm *(Spodoptera frugiperda)*, corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays)*.

In another aspect, the present invention also relates to the use of a genome editing system according to any of the embodiments described above.

In a preferred embodiment, the use is for conferring or increasing resistance or tolerance to fall armyworm *(Spodoptera frugiperda)* and, optionally to a fungal pathogen, in particular *Fusarium species,* to/in a plant, preferably a maize *(Zea mays)* plant.

In one embodiment of the use described above, the at least one genome editing system comprises

(a) at least one site-specific nuclease or site-specific nickase, and optionally, in case a CRISPR system is used, at least one guide molecule or a sequence encoding the same, and
(b) optionally, at least one repair template, or a sequence encoding the same,

optionally, wherein the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or wherein the at least one genome editing system is selected from a zinc finger nuclease system, or a transcription activator-like nuclease system, or a meganuclease system, or any combination, variant, or an active fragment thereof.

In another embodiment of the use described in any of the embodiments above, the genome editing system comprises a protospacer having a nucleic acid sequence of SEQ ID NO: 32, 33, 34 or 35, or a nucleic acid sequence having 85%, 86%, 87%, 88%, 89%, 90%, 91%, 92%, 93%, 94%, 95%, 96%, 97%, 98% or at least 99% to the sequence of any of SEQ ID NOs: 32, 33, 34 or 35.

In one embodiment of the use described above, the genome editing system targets the endogenous Lox3 gene in a maize plant, wherein the genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same

for conferring or increasing resistance or tolerance to one or more insect(s) selected from the group consisting of fall army worm *(Spodoptera frugiperda)*, corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays)*.

In one embodiment of the use described above, the genome editing system comprises a protospacer having a nucleic acid sequence of SEQ ID NO: 32, 33, 34 or 35, or a nucleic acid sequence having 85%, 86%, 87%, 88%, 89%, 90%, 91%, 92%, 93%, 94%, 95%, 96%, 97%, 98% or at least 99% to the sequence of any of SEQ ID NOs: 32, 33, 34 or 35.

In another embodiment of the use described above, the genome editing system targets the endogenous Lox3 gene in an oilseed rape plant, wherein the genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same

for conferring or increasing resistance or tolerance to one or more insect(s) selected from the group consisting of green peach aphid *(Myzus persicae),* diamondback moth *(Plutella xylostella),* cabbage stem flea beetle *(Psylliodes chrysocephala),* crucifer flea beetle *(Phyllotreta cruciferae),* striped flea beetle *(Phyllotreta striolata),* hop flea beetle *(Psylliodes punctulata),* rape stem weevil *(Ceutorhynchus picitarsis)* and cabbage stem weevil *(Ceutorhynchus quadridens)* and, optionally conferring or increasing resistance or tolerance to one or more fungal pathogen(s) selected from the group consisting of *Phoma lingam* and *Plasmodiophora brassicae* to/in oilseed rape *(Brassica napus).*

The present invention also relates to the use of an expression construct encoding a genome editing system, which targets the endogenous Lox3 gene in a maize plant, wherein the genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same,

wherein the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or wherein the at least one genome editing system is selected from a zinc finger nuclease system, or a transcription activator-like nuclease system, or a meganuclease system, or any combination, variant, or an active fragment thereof for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus)* and, optionally conferring or increasing resistance or tolerance to one or more fungal pathogen(s) selected from the group consisting of *Fusarium species, Colletotrichum species,* in particular *Colletotrichum graminicola* and *Colletotrichum sublineolum, Diplodia species, Cercospora zeina* and *Cercospora zeae-maydis* to/in maize *(Zea mays).*

Furthermore, the present invention also relates to a use of an expression construct encoding a genome editing system, which targets the endogenous Lox3 gene in an oilseed rape plant, wherein the genome editing system comprises

(a) at least one site-specific nuclease or site-specific nickase, and optionally, in case a CRISPR system is used, at least one guide molecule or a sequence encoding the same, and

(b) optionally, at least one repair template, or a sequence encoding the same,

wherein the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or wherein the at least one genome editing system is selected from a zinc finger nuclease system, or a transcription activator-like nuclease system, or a meganuclease system, or any combination, variant, or an active fragment thereof

for conferring or increasing resistance or tolerance to one or more insect(s) selected from the group consisting of green peach aphid *(Myzus persicae),* diamondback moth *(Plutella xylostella),* cabbage stem flea beetle *(Psylliodes chrysocephala),* crucifer flea beetle *(Phyllotreta cruciferae),* striped flea beetle *(Phyllotreta striolata),* hop flea beetle *(Psylliodes punctulata),* rape stem weevil *(Ceutorhynchus picitarsis)* and cabbage stem weevil *(Ceutorhynchus quadridens)* and, optionally conferring or increasing resistance or tolerance to one or more fungal pathogen(s) selected from the group consisting of *Phoma lingam* and *Plasmodiophora brassicae* to/in oilseed rape *(Brassica napus).*

In one embodiment of the use described above, the expression construct comprises a crRNA encoded by a nucleic acid sequence of any of SEQ ID NOs: 46 to 49, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of any of SEQ ID NO: 46 to 49.

In another embodiment of the use described above, the genome editing system is encoded by a plasmid of the nucleic acid sequence of SEQ ID NO: 50 or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 50.

The present invention also relates to the use a vector encoding an genome editing system as defined above for conferring or increasing resistance or tolerance to one or more insect(s) selected from the group consisting of fall army worm *(Spodoptera frugiperda),* corn leafhopper *(Dalbulus maidis)* and green belly stink bug *(Dichelops melacanthus) and, optionally* conferring or increasing resistance or tolerance to one or more fungal pathogen(s) selected from the group consisting of *Fusarium species, Colletotrichum species,* in particular *Colletotrichum graminicola* and *Colletot-*

*richum sublineolum, Diplodia species, Cercospora zeina* and *Cercospora zeae-maydis* to/in maize *(Zea mays).*

Finally, the present invention also relates to the use of a vector encoding an genome editing system as defined above for conferring or increasing resistance or tolerance to one or more insect(s) selected from the group consisting of green peach aphid *(Myzus persicae),* diamondback moth *(Plutella xylostella),* cabbage stem flea beetle *(Psylliodes chrysocephala),* crucifer flea beetle *(Phyllotreta cruciferae),* striped flea beetle *(Phyllotreta striolata),* hop flea beetle *(Psylliodes punctulata),* rape stem weevil *(Ceutorhynchus picitarsis)* and cabbage stem weevil *(Ceutorhynchus quadridens)* and, optionally conferring or increasing resistance or tolerance to one or more fungal pathogen(s) selected from the group consisting of *Phoma lingam* and *Plasmodiophora brassicae* to/in oilseed rape *(Brassica napus).*

**Example 1: Generation of RNAi corn lines with Lox3 knock-down**

An RNAi hairpin construct directed against the ZmLox3 gene was constructed (Figure 1, SEQ ID NOs: 1 to 5) and transformed into corn genotype A188 by *Agrobacterium tumefaciens* mediated transformation. Transgenic T0 plants were identified by PCR and transferred into the greenhouse for T1 seed production by selfing. A segregation analysis was performed with T1 plants. Homozygous ZmLox3_RNAi plants and non-transgenic, azygous plants were selected and T2 seeds were produced. The T2 seeds were used for the insect resistance assays.

Downregulation of ZmLox3 gene (SEQ ID NO: 6 - 16, 87-89) in the ZmLox3_RNAi lines was shown by qRT-PCR after isolation of RNA from leaves of homozygous GM and non-transgenic, azygous plants (Figure 2). The expression of ZmLox3 was measured using the primer S3460 (SEQ ID NO: 18) and primer S3461 (SEQ ID NO: 19). Lox3 expression was normalized against the expression of the housekeeping gene Elongation factor 1-alpha (EF1, SEQ ID NO: 22 and 23) using the primer S3428 (SEQ ID NO: 20) and the primer S3429 (SEQ ID NO: 21).

The expression analysis of corn leaves revealed that the lines FDC003-T002, FDC003-T005, FDC003-T010, FDC003-T021 and FDC003-T023 show a strong reduction of Lox3 expression (Figure 2).

**Example 2: Determine insect resistance by measuring larval weight of fall army worm feeding on Lox3 RNAi lines**

The purpose of this experiment is to evaluate if Lox3 gene down-regulation is linked to fall armyworm tolerance in corn. To test this, fall armyworm *(Spodoptera frugiperda)* larval performance was chosen as a measure of antibiosis-based resistance.

Three ZmLox3_RNAi corn lines (FDC003-T002, FDC003-T011 and FDC003-T023) were evaluated using unmodified A188 corn as control. Three independent insect resistance experiments were performed. For each experiment, five biological replicates were used for A188 and for each ZmLox3_RNAi lines. Three newly hatched fall armyworm larva were added into individual Falcon tubes containing corn leaves. Fresh leaves were provided daily and after ten days the larva weight was scored.

All tested lines conferred a significant increase of resistance against fall armyworm (Figure 3). These data showed that the ZmLox3 gene is a susceptibility gene for fall armyworm and that down-regulation of ZmLox3 expression will improve resistance.

**Example 3: Determine fungal resistance of Lox3 RNAi lines**

Since stalk and ear rot caused by *Fusarium spp.* are favored by the mechanical damage caused by invading insects, the Lox3 mediated insect resistance may have an effect on fungal resistance.

The insect resistant line FDC003-T023 was crossed with tester line RP5G and compared with test crosses of FDC0003-T029 and FDC0003-T029 azygous with tester line RP5G, which did not show LOX3 reduction as determined by qRT-PCR (Figure 4, left). The FDC003-T023 hybrid showed 13 and 8 % less *Fusarium* infection compared to the FDC0003-T029 and FDC0003-T029 azygous hybrids (Figure 4, right), which revealed that an insect resistant RNAi line also has higher fungal resistance.

**Example 4: Generation of Lox3 SDN1 knock-down in tropical corn by genome editing**

Lox3 was knocked out in tropical corn genotypes by means of a site-directed nuclease.

1. Tropical corn lines were grown in soil from seed for five weeks and immature tassels were harvested as previously described (WO2021170785).

2. Tassel material was bombarded using the following vectors:

    a. TGCD035 (ZmPLT5, SEQ ID Nos: 24 and 25)

    b. GEZM145 (RBP8, SEQ ID Nos: 26 and 27)

c. GEZM152 (gRNA, protospacer m7GEP336:
GCACGTTCTTGCGCATGAGCA, SEQ ID NO: 32)

d. GEZM153 (gRNA, protospacer m7GEP337:
GCGCCACCGTCGTTGACAGCA, SEQ ID NO: 33)

e. GEZM154 (gRNA, protospacer m7GEP338:
CTGTGCAGACAACGGCAACCG, SEQ ID NO: 34)

f. GEZM155 (gRNA, protospacer m7GEP339:
AGCTTCTCCACCTCCCAGTCG, SEQ ID NO: 35)

g. GEMT121 (LbCpf1-RR and tdTomato, SEQ ID Nos: 28 - 31)

3. Using the standard regeneration protocol for tropical corn lines, 12 plants were sampled and analyzed by amplicon sequencing using the following primers:

**Table 1:**

| Primer | Sequence | SEQ ID NO |
|---|---|---|
| 946R | GCTCTCTCGGCCCCCACTTTTT | 71 |
| 1145F | TGTTCTGCGCCCAGGCTACA | 72 |
| 346F | TCGCGAACACACCGGTCGTA | 73 |
| 1948R | ACTCACATGCCTCGCCCTCA | 74 |

Three plants were identified with edits in the Lox3 gene. All three plants were edited in multiple locations (Table 2).

**Table 2:**

| | Target 1 | Target 2 | Target 3 |
|---|---|---|---|
| GEZM152T012 | 100%: 3&12 bp deletions | 100%: 3&12 bp deletions | 50%: 15 bp deletion |
| GEZM152T013 | 50%: 5 bp deletion | 50%: 5 bp deletion | 50%: 23 bp deletion |
| GEZM152T014 | 100%: 3 & 11 bp deletions | 100%: 3 & 11 bp deletions | 50%: 15 bp deletion |

4. Plants were transferred from media to soil and kept in short day conditions (in a lean-in Conviron) for as long as possible to trigger tassel and ear development before ultimately moving them to standard greenhouse conditions.

**Example 5: Determine tropical corn insect resistance by measuring larval weight of fall armyworm feeding on Lox3 SDN1 tropical corn edited lines**

The purpose of this experiment is to evaluate if Lox3 gene knock-out is linked to fall armyworm tolerance in tropical corn (SL57). To test this, fall armyworm (*Spodoptera frugiperda*) larval performance was chosen as a measure of antibiosis-based resistance.

Three ZmLox3_SDN-1 tropical corn lines (GEZM152T012, GEZM152T013, GEZM152T014) were evaluated using unmodified SL57 tropical corn as control. Three independent insect resistance experiments were performed. For each experiment, five biological replicates were used for the unmodified SL57 tropical corn and for each ZmLox3_SDN-1 lines. Three newly hatched fall armyworm larva were added into individual Falcon tubes containing corn leaves. Fresh leaves were provided daily and after ten days the larva weight was scored.

**Example 6: Generation of Lox3 TILLING mutants**

**EMS mutagenesis:** A mutagenized population (EMS treatment) from PH207 was developed. The exonic region 7 of Zm00008a004913 was screened and 5 positive mutants harboring amino acid changes and stop codons were detected (SEQ ID NOs: 36 - 40, Table 3). In Zm00008a004913 cDNA of Mutant WVP18-09344-014 C at position 1876 replaced by T (see also SEQ ID NO: 43), leading to an amino acid exchange from P to S at position 569 (see also SEQ ID NO: 37); in Zm00008a004913 cDNA of Mutant WVP18-09309-014 and WVP18-09339-009 G at position 2004 replaced by A (see also SEQ ID NO: 44), leading to an amino acid exchange from W to STOP at position 668 (see also SEQ ID NO: 39); in Zm00008a004913 cDNA of Mutant WVP18-09358-016 G at position 2079 replaced by A (see also SEQ ID NO: 42), leading to an amino acid exchange from W to STOP at position 693 (see also SEQ ID NO: 37); in Zm00008a004913 cDNA of Mutant WVP18-09307-014 G at position 2287 replaced by A (see also SEQ ID NO: 45), leading to an amino acid exchange from G to S at position 763 (see also SEQ ID NO: 40). After selfing of these mutants to produce two homozygote classes (homozygote wildtype and mutant), they have been evaluated in the field for resistance to two *Fusarium species* (*F. graminearum, F. verticillioides,* see Example 7).

**Table 3:**

| EternalName | Gen-Name | Code | Pos CDS | Base WT | Base Mut | Position in AS sec | | AS Mut |
|---|---|---|---|---|---|---|---|---|
| WVP18-09344-014 | LOX3 | PH207m034b | 1876 | C | T | 569 | P | S |
| WVP18-09309-014 | LOX3 | PH207m034d | 2004 | G | A | 668 | W | STOP |
| *WVP18-09339-009* | *LOX3* | *PH207m034d* | 2004 | G | A | *668* | *W* | *STOP* |
| WVP18-09358-016 | LOX3 | PH207m034f | 2079 | G | A | 693 | W | STOP |
| WVP18-09307-014 | LOX3 | PH207m034h | 2287 | G | A | 763 | G | S |

**Example 7: Determine fungal resistance of Lox3 TILLING mutants**

After selfing of these mutants to produce two homozygote classes (homozygote wildtype and mutant), they have been evaluated in the field for resistance to two *Fusarium species* (*F. graminearum* (FG), *F. verticilloides* (FV)).

In 2020 plants were grown in rows with 20 plants per row in the location GON and in 2021 in MUR. Ten plants of each experimental unit were inoculated four to six days after the experimental unit was flowering, excluding the first plant of the row to avoid possible border effect. Rows were declared as flowering when at least 50% of the plants in the row presented extruded silks. Female flowering (FF) dates were collected for each row daily.

1 ml of an inoculum suspension with a concentration of 15.000 conidia ml$^{-1}$ (*F. graminearum* (FG)) and 1.000.000 conidia ml$^{-1}$ (*F. verticilloides* (FV)) was inoculated into the maize silk channel with a self-refilling syringe. The experiments for the two different species were kept separate.

Approximately 50 days after inoculation, cobs were dehusked and all plants were visually assessed for *Fusarium* symptoms by estimating the percentage of the ear covered by mycelium. The remnant not-inoculated plants of each experimental unit were used as a control of the proportion of naturally infected cobs. The arithmetic mean of the 10 assessed inoculated were employed for further analyses.

The homozygous mutant class for PH207m034b and PH207m034h showed lower infection of *F. graminearum* (FG) and *F. verticilloides* (FV) (Table 4).

**Table 4:**

| Entry Name | FV_GON | FG_GON | FV_MUR | FG_MUR | Gene | Plant_2020 | Gene | Plant_2021 | mean_FV_GON (2020) | mean_FG_GON (2020) | mean_FV_MUR (2021) | mean_FG_MUR (2021) |
|---|---|---|---|---|---|---|---|---|---|---|---|---|
| PH207 | 12,60 | 41,67 | 45,50 | 38,13 | | WVP20-73701/MIX | | WVP20-72327/MIX | | | | |
| PH207 | 18,67 | 30,00 | 58,50 | 32,78 | | WVP20-73701/MIX | | WVP20-72372/MIX | | | 43,92 | 35,36 |
| PH207 | 37,50 | 56,00 | 42,22 | 38,89 | | WVP20-73701/MIX | | WVN19-79936/MIX | 22,92 | 42,56 | | |
| PH207m034b | | | 16,11 | 8,86 | LOX3-MUT | WVP20-73214/005 | LOX3-MUT | WVP20-73212/007 | | | | |
| PH207m034b | 17,40 | | 35,50 | 12,50 | LOX3-MUT | WVP20-73216/005 | LOX3-MUT | WVP20-73212/007 | | | | |
| PH207m034b | 18,33 | 0,00 | 8,00 | 4,11 | LOX3-MUT | WVP20-73217/003 | LOX3-MUT | WVP20-73212/007 | | | 20,65 | 8,70 |
| PH207m034b | 9,60 | 10,00 | 23,00 | 9,33 | LOX3-MUT | WVP20-73217/006 | LOX3-MUT | WVP20-73212/007 | 15,11 | 5,00 | | |
| PH207w034b | 36,50 | 9,63 | 14,17 | 25,00 | LOX3-WT | WVP20-73214/006 | LOX3-WT | WVP20-73212/007 | | | | |
| PH207w034b | 40,00 | 56,63 | 28,89 | | LOX3-WT | WVP20-73215/005 | LOX3-WT | WVP20-73212/007 | | | | |
| PH207w034b | 19,00 | 30,00 | 24,00 | 1,50 | LOX3-WT | WVP20-73217/004 | LOX3-WT | WVP20-73212/007 | 26,13 | 33,23 | | |
| PH207w034b | 9,00 | 36,67 | | 11,50 | LOX3-WT | WVP20-73217/007 | LOX3-WT | WVP20-73212/007 | | | 20,51 | 16,58 |
| PH207w034b | | | 15,00 | 28,33 | | | LOX3-WT | WVP20-73212/007 | | | | |
| PH207m034h | 34,44 | 53,50 | n.d. | n.d. | LOX3-MUT | WVP20-73264/004 | | | | | | |
| PH207m034h | 28,50 | 50,00 | n.d. | n.d. | LOX3-MUT | WVP20-73266/004 | | | | | | |
| PH207m034h | 23,00 | 25,00 | n.d. | n.d. | LOX3-MUT | WVP20-73268/001 | | | 28,44 | 45,72 | | |
| PH207m034h | 27,80 | 54,38 | n.d. | n.d. | LOX3-MUT | WVP20-73270/002 | | | | | | |
| PH207w034h | 57,00 | 46,80 | n.d. | n.d. | LOX3-WT | WVP20-73267/002 | | | | | | |

| Entry Name | FV_GON | FG_GON | FV_MUR | FG_MUR | Gene | Plant_2020 | Gene | Plant_2021 | mean_FV_GON | mean_FG_GON | mean_FV_MUR | mean_FG_MUR |
|---|---|---|---|---|---|---|---|---|---|---|---|---|
| PH207w0 34h | 28,11 | 28,00 | n.d. | n.d. | LOX3-WT | WVP20-73267/003 | | | | | | |
| PH207w0 34h | 37,78 | 71,67 | n.d. | n.d. | LOX3-WT | WVP20-73268/002 | | | | | | |
| PH207w0 34h | 32,40 | 45,50 | n.d. | n.d. | LOX3-WT | WVP20-73270/004 | | | 38,82 | 47,99 | | |
| PH207m0 34d | n.d. | n.d. | 0,00 | 65,83 | | | LOX3-MUT | WVN18-76855/003 | n.d. | n.d. | | |
| PH207m0 34d | n.d. | n.d. | 12,50 | | | | LOX3-MUT | WVN18-76855/008 | n.d. | n.d. | | |
| PH207m0 34d | n.d. | n.d. | | 40,00 | | | LOX3-MUT | WVN18-76856/005 | n.d. | n.d. | | |
| PH207m0 34d | n.d. | n.d. | 57,50 | 15,00 | | | LOX3-MUT | WVN18-76856/012 | n.d. | n.d. | | |
| PH207m0 34d | n.d. | n.d. | 40,56 | 41,43 | | | LOX3-MUT | WVN18-76857/009 | n.d. | n.d. | | |
| PH207m0 34d | n.d. | n.d. | 19,38 | 47,50 | | | LOX3-MUT | WVN18-76858/013 | n.d. | n.d. | 25,99 | 41,95 |
| PH207w0 34d | n.d. | n.d. | 100,00 | 53,75 | | | LOX3-WT | WVN18-76854/012 | n.d. | n.d. | | |
| PH207w0 34d | n.d. | n.d. | 7,50 | 31,67 | | | LOX3-WT | WVN18-76856/006 | n.d. | n.d. | | |
| PH207w0 34d | n.d. | n.d. | 71,67 | 51,67 | | | LOX3-WT | WVN18-76856/009 | n.d. | n.d. | | |
| PH207w0 34d | n.d. | n.d. | 57,00 | 45,00 | | | LOX3-WT | WVN18-76857/007 | n.d. | n.d. | | |
| PH207w0 34d | n.d. | n.d. | 41,67 | 46,67 | | | LOX3-WT | WVN18-76858/008 | n.d. | n.d. | 55,57 | 45,75 |

EP 4 215 039 A1

34

**Example 8: Determine insect resistance of Lox3 TILLING mutants**

The purpose of this experiment is to evaluate if Lox3 Tilling stop-mutations is linked to fall armyworm tolerance in temperate corn (line PH207). To test this, fall armyworm *(Spodoptera frugiperda)* larval performance was chosen as a measure of antibiosis-based resistance.

Two different Stop-Mutants for Lox3, the according wild-types derived from a heterozygote mutant plant and the line PH207 which is the parent of the mutant population were evaluated. Three independent insect resistance experiments were performed and for each experiment, five biological replicates were used. Three newly hatched fall armyworm larva were added into individual Falcon tubes containing corn leaves. Fresh leaves were provided daily and after ten days the larva weight was scored.

**Example 9: Generation of Lox3 SDN1 knock-down in OSR by genome editing**

1. Agro-based transformation, nuclease is provided in the same construct together with the gRNAs (Figure 5: construct map, plasmid sequence: pZFNnptII-LbCpf1-tDT-lox3_TTTV, SEQ ID NO: 50); four crRNAs were used in total to target the OSR lox3 gene (SEQ ID Nos: 46-49); BnLox3 sequences SEQ ID Nos: 75-86.

a. Agro-based transformation was adopted to generate GE plants using various different starting materials of seedlings between 1 to 10 days old.

The oil seed rape transformation protocol includes the following steps:

1. Seed sterilization with 70% ethanol and bleach

2. Seed germination on germination medium

3. *Agrobacterium* culture of a strain containing the construct of interest

4. Preparation of *Agrobacterium* for inoculation

5. Preparation of plant material and co-culture

6. Recovery of callus

7. Selection

8. Shoot growth

9. Rooting

2. Molecular analyses: Next Generation Sequencing (NGS), digital droplet PCR (ddPCR) and Decomposition Regression to Identify Variations for Editing Events (DRIVE)
a. The SDN-1 gene edited plants were first analyzed for the presence of the transgene by qPCR using the settings shown in tables 5 and 6:

**Table 5:**

|  | primer | work stock concentration (uM) | SEQ ID NO |
|---|---|---|---|
| Primers | cruaxxxxxxf02x | 20 | 51 |
|  | cruaxxxxxxr01x | 20 | 52 |
|  | nptIIxxxf01 | 20 | 53 |
|  | nptIIxxxr01 | 20 | 54 |
|  | tDTxxxf04 | 20 | 55 |
|  | tDTxxxr01 | 20 | 56 |
| probes | CruaxxxMGB | 10 | 57 |
|  | nptIIxxxMGB | 10 | 58 |
|  | tDTxxxMGB | 10 | 59 |

qPCR parameters:

**Table 6:**

| 50°C | 2min |  |
|---|---|---|
| 95°C | 5min |  |
| 95°C | 15s | 40cycles |
| 60°C | 30s | |

b. The transgenic plants were then analyzed by qPCR using the primers sets SEQ ID 60, 61 and 62 (probe) for nuclease test.

c. The editing profile was then obtained by amplifying and sequencing the PCR product using the primers sets as shown in table 7 and then run Drive analysis on OMICS to get editing information.

**Table 7:**

|  | Primer | SEQ ID NO: |
|---|---|---|
| IR106_lox3_F1 | CCTCTGACCTCCAAAAGACCCTTA | 63 |
| IR106_lox3_F2 | CCATTACTTGTTTGGTCGGCGT | 64 |
| IR106_lox3_F3 | GCCATCACTTGTTTTCTCGGC | 65 |
| IR106_lox3_F4 | CCGTCACTTGTTTTCTCGGC | 66 |
| IR106_lox3_R1 | TCAAAGGCTAATATAACTGACACGT | 67 |
| IR106_lox3_R2 | GGCAAACGCGTTTCCTTAATTCA | 68 |
| IR106_lox3_R3 | ATAACGCTGATGTGCTAAGC | 69 |
| IR106_lox3_R4 | GTTAATAACGCTGATGTACTAAGC | 70 |

**Example 10: Determine OSR insect resistance by measuring leaf damage from diamondback moth and by measuring aphid reproduction from insect feeding on Lox3 SDN1 edited line**

The purpose of this experiment is to evaluate if Lox3 gene knock-out is linked to diamondback moth and green peach aphid tolerance in OSR (Palma). To test this, diamondback moth *(Plutella xylostella)* feeding damage and green peach aphid *(Myzus persicae)* reproduction were chosen as a measure of plant resistance.

One Lox3_SDN-1 OSR line was evaluated using unmodified Palma OSR as control. Three independent insect resistance experiments were performed and for each experiment, fourteen biological replicates were used.

For the test using diamondback moth, four newly hatched larva were placed on each one-week-old OSR seedlings. Four days later, the percentage of plant damage (= percentage of leaf eaten by the larva) was visually scored using a 0 to 100% scale.

For the test using green peach aphid, one adult aphid was placed on each one-week-old OSR seedlings. Seven days later, the number of aphids on each seedling was recorded (adult aphid + progeny).

**Example 11: Determine fungal resistance of Lox3 edited lines**

**Clubroot test**

Infected oil seed rape roots are collected in the field. 100 g of infected roots are crushed with 400 ml water. The solution is then roughly filtered. Two different isolates were used in the screening, isolate A (Mendel-based varieties show still resistance) and C (Mendel-based varieties are highly susceptible).

OSR plants to be tested were sown in sand and were picked after 10 to 14 days. Roots were washed and incubated in the spore solution for 15 to 20 minutes. Plants were then removed and planted into a soil/sand substrate. Afterwards the spore solution was additionally pipetted directly on to the plants. Afterwards they were grown under greenhouse conditions for approximately 6 weeks.

After washing of the roots, the gall formation was scored from 1, meaning no gall formation to 4 strong gall formation (Fig. 6). A disease index (DI) was calculated (E. Diederichsen and M.D. Sacristan, Plant Breeding 115, 5-10 (1996), Disease response of resynthesized *Brassica napus* L. lines carrying different combinations of resistance to *Plasmodiophora brassicae* Wor.) by using the following formula:

$$DI = \frac{\sum(0n_0 + 1n_1 + 2n_2 + 3n_3)}{3N} \times 100$$

The values n0 to n3 correspond to the number of plants per class and N being the total number of tested plants. Genotypes with a DI<10 were assessed to be highly resistant. A DI between 10-25 leads to an assessment as resistant and genotypes with a DI>25 are rated as susceptible.

**Blackleg test**

**Claims**

1. A method for conferring or increasing resistance or tolerance to fall armyworm *(Spodoptera frugiperda)* and, optionally to a fungal pathogen, in particular *Fusarium species,* to/in a plant, preferably a maize *(Zea mays)* plant, comprising the steps of:

   (i) providing at least one plant cell, preferably a maize plant cell;
   (ii) introducing into the at least one plant cell at least one gene silencing construct, at least one genome editing system or a genome modification, which leads to a targeted knock-down or a knock-out of a Lox3 gene endogenous to the plant;
   (iii) obtaining at least one modified plant cell having reduced or abolished expression of the Lox3 gene; and
   (iv) obtaining at least one plant cell, tissue, organ, plant or seed having reduced or abolished expression of the Lox3 gene, optionally after an additional step of regenerating the plant tissue, organ, plant or seed from the at least one modified cell.

2. The method according to claim 1, wherein the Lox3 gene is represented by a nucleic acid sequence of SEQ ID NO: 6, 7, 9, 10, 12, 13, 15, 16, 87 or 88, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 6, 7, 9, 10, 12, 13, 15, 16, 87 or 88, respectively.

3. The method according to claim 1 or 2, wherein the Lox3 gene encodes an amino acid sequence of SEQ ID NO: 8, 11, 14, 17 or 89 or an amino acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 8, 11, 14, 17 or 89.

4. The method according to any of the preceding claims, wherein in step (ii) a construct is introduced into the at least one plant cell, which targets the Lox3 gene for gene silencing wherein the construct is or wherein the construct encodes an RNAi construct comprising a sense and an antisense sequence targeting the Lox3 gene, the RNAi construct forming an RNA hairpin upon transcription,

(a) wherein the sense sequence is encoded by a nucleic acid sequence of SEQ ID NO: 1, or a nucleic acid sequence having a sequence identity of at least 90%, 91%, 92%, 93%, 94%, 95%, 96%, 97%, 98% or at least 99% to the sequence of SEQ ID NO: 1; and/or
(b) wherein the antisense sequence is encoded by a nucleic acid sequence of SEQ ID NO: 2, or a nucleic acid sequence having a sequence identity of at least 90%, 91%, 92%, 93%, 94%, 95%, 96%, 97%, 98% or at least 99% to the sequence of SEQ ID NO: 2, and/or
(c) preferably wherein the construct comprises a nucleic acid sequence of SEQ ID NO: 4, or a nucleic acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4, optionally wherein the construct is introduced into the at least one plant cell by transformation or transfection mediated by biolistic bombardment, *Agrobacterium*-mediated transformation, micro- or nanoparticle delivery, chemical transfection, or a combination thereof.

5. The method according to claim 4, wherein the RNA hairpin has an intervening intron loop sequence comprising a nucleic acid sequence of SEQ ID NO: 3, or a nucleic acid sequence having a sequence identity of at least 85%, 86%, 87%, 88%, 89%, 90%, 91%, 92%, 93%, 94%, 95%, 96%, 97%, 98% or at least 99% to the sequence of SEQ ID NO: 3.

6. The method according to any of the preceding claims, wherein in step (ii) a construct is introduced into the at least one plant cell, which targets the Lox3 gene for gene silencing, preferably wherein the construct is introduced as part of a vector the vector being introduced into the plant cell, which vector comprises or consists of a nucleic acid sequence of SEQ ID NO: 5, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 5.

7. The method according to any of claims 1 to 3, wherein in step (ii) at least one genome editing system is introduced into the at least one cell, which construct targets the Lox3 gene, wherein the at least one genome editing system comprises

(a) at least one site-specific nuclease or site-specific nickase, and optionally, in case a CRISPR system is used, at least one guide molecule or a sequence encoding the same, and
(b) optionally, at least one repair template, or a sequence encoding the same, optionally, wherein the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or wherein the at least one genome editing system is selected from a zinc finger nuclease system, or a transcription activator-like nuclease system, or a meganuclease system, or any combination, variant, or an active fragment thereof; and

optionally, wherein the at least one genome editing system is introduced into the at least one maize cell by transformation or transfection mediated by biolistic bombardment, *Agrobacterium*-mediated transformation, micro- or nanoparticle delivery, chemical transfection, or a combination thereof.

8. The method according to any of claims 1 to 3, wherein in step (ii) a mutagenesis is performed to obtain at least one genome modification on a single or on a plurality of cell(s) by applying chemicals or radiation, preferably wherein an alkylating agent, including ethyl methanesulfonate is applied to the single or the plurality of cell(s) to induce mutagenesis.

9. The method according to claim 8, wherein one or more mutations in the Lox3 gene are inserted and identified by TILLING in step (ii) and/or wherein one or more cell(s) with knock-down or knock-out mutations in the Lox3 gene are selected in step (ii).

10. A maize cell, maize tissue, maize organ, maize plant or maize seed obtained or obtainable by a method according to any of the preceding claims.

11. An expression construct, which targets the Lox3 gene in maize for gene silencing, wherein the construct encodes an RNAi construct comprising a sense and an antisense sequence targeting the Lox3 gene endogenous to a maize plant, which RNAi construct forms an RNA hairpin upon transcription, wherein

(a) the sense sequence is encoded by a nucleic acid sequence of SEQ ID NO: 1, or a nucleic acid sequence having a sequence identity of at least 90%, 91%, 92%, 93%, 94%, 95%, 96%, 97%, 98% or at least 99% to the sequence of SEQ ID NO: 1; and/or
(b) wherein the antisense sequence is encoded by a nucleic acid sequence of SEQ ID NO: 2, or a nucleic acid sequence having a sequence identity of at least 90%, 91%, 92%, 93%, 94%, 95%, 96%, 97%, 98% or at least 99% to the sequence of SEQ ID NO: 2, and/or optionally wherein the RNA hairpin has an intervening intron loop sequence comprising a nucleic acid sequence of SEQ ID NO: 3, or a nucleic acid sequence having a sequence identity of at least 85%, 86%, 87%, 88%, 89%, 90%, 91%, 92%, 93%, 94%, 95%, 96%, 97%, 98% or at least 95% to the sequence of SEQ ID NO: 3; and/or
(c) preferably wherein the construct comprises a nucleic acid sequence of SEQ ID NO: 4, or a nucleic acid sequence having a sequence identity of at least 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4.

12. An RNAi hairpin construct for conferring or increasing resistance or tolerance to fall armyworm *(Spodoptera frugiperda)* and, optionally to a fungal pathogen, in particular *Fusarium species,* to/in a maize plant *(Zea mays),* wherein the RNAi hairpin construct comprises a nucleic acid sequence of SEQ ID NO: 4, or a nucleic acid sequence having a sequence identity of at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or at least 99% to the sequence of SEQ ID NO: 4.

13. An expression construct encoding a genome editing system, which targets the Lox3 gene in maize, wherein the genome editing system comprises

(a) at least one site-specific nuclease or site-specific nickase, and optionally, in case a CRISPR system is used, at least one guide molecule or a sequence encoding the same, and
(b) optionally, at least one repair template, or a sequence encoding the same,

preferably wherein the at least one genome editing system is selected from a CRISPR/Cas system, preferably from a CRISPR/MAD7 system, a CRISPR/Cpf1 (CRISPR/Cas12a) system, a CRISPR/MAD2 system, a CRISPR/Cas9 system, a CRISPR/CasX system, a CRISPR/CasY system, a CRISPR/Cas13 system, or a CRISPR/Csm system, or wherein the at least one genome editing system is selected from a zinc finger nuclease system, or a transcription activator-like nuclease system, or a meganuclease system, or any combination, variant, or an active fragment thereof.

14. A maize cell, maize tissue, maize organ, maize plant or maize seed comprising an expression construct, or a vector encoding the same, or an RNAi hairpin construct, or a vector encoding the same, according to any of claims 11 to 13.

15. A use of at least one gene silencing construct and/or of at least one genome editing system and/or of at least one genome modification as defined in any one of claims 1 to 9 or 11 to 13, which leads to a targeted knock-down or a knock-out of the endogenous a Lox3 gene, for conferring or increasing resistance or tolerance to fall armyworm *(Spodoptera frugiperda)* and, optionally to a fungal pathogen, in particular *Fusarium species,* to/in a plant, preferably a maize *(Zea mays)* plant.

**Figure 1:**

**Figure 2:**

**Figure 3:**

**Figure 4:**

**Figure 5:**

**Figure 6:**

**EUROPEAN SEARCH REPORT**

Application Number

EP 22 15 3142

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2021/207148 A1 (PAIRWISE PLANTS SERVICES INC [US]) 14 October 2021 (2021-10-14) * examples 1,5 * * page 56, line 18 – page 57, line 7 * | 10,11, 13,14 | INV. A01H1/00 C12N9/02 C12N15/82 |
| X | PATHI KRISHNA MOHAN ET AL: "Engineering Smut Resistance in Maize by Site-Directed Mutagenesis of LIPOXYGENASE 3", FRONTIERS IN PLANT SCIENCE, vol. 11, 21 October 2020 (2020-10-21), XP55939294, DOI: 10.3389/fpls.2020.543895 * the whole document * | 10,11, 13,14 | |
| X | Gao Xiquan ET AL: "922 / Molecular Plant-Microbe Interactions Disruption of a Maize 9-Lipoxygenase Results in Increased Resistance to Fungal Pathogens and Reduced Levels of Contamination with Mycotoxin Fumonisin", , vol. 8 1 January 2007 (2007-01-01), pages 922-933, XP055819789, Retrieved from the Internet: URL:https://apsjournals.apsnet.org/doi/abs /10.1094/MPMI-20-8-0922?url_ver=Z39.88-200 3&rfr_id=ori:rid:crossref.org&rfr_dat=cr_p ub%20%200pubmed [retrieved on 2021-06-30] * the whole document * | 10,11 | TECHNICAL FIELDS SEARCHED (IPC) A01H C12N |

-/--

The present search report has been drawn up for all claims

| Place of search | Date of completion of the search | Examiner |
|---|---|---|
| Munich | 7 July 2022 | Kania, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

**Application Number**

EP 22 15 3142

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | BAI SUYANG ET AL: "Knock-down of OsLOX by RNA interference leads to improved seed viability in rice", JOURNAL OF PLANT BIOLOGY, BOTANICAL SOCIETY OF KOREA, SEOUL, KR, vol. 58, no. 5, 2 October 2015 (2015-10-02), pages 293-302, XP035551769, ISSN: 1226-9239, DOI: 10.1007/S12374-015-0133-6 [retrieved on 2015-10-02] | 1-15 | |
| A,D | Gao Xiquan ET AL: "Maize 9-Lipoxygenase ZmLOX3 Controls Development, Root-Specific Expression of Defense Genes, and Resistance to Root-Knot Nematodes", Molecular Plant-Microbe Interactions, vol. 21 1 January 2008 (2008-01-01), pages 98-109, XP55939304, Retrieved from the Internet: URL:https://apsjournals.apsnet.org/doi/abs/10.1094/MPMI-21-1-0098?url_ver=Z39.88-2003&rfr_id=ori:rid:crossref.org&rfr_dat=cr_pub%20%200pubmed [retrieved on 2022-07-06] | 1-15 | TECHNICAL FIELDS SEARCHED (IPC) |

-/--

The present search report has been drawn up for all claims

| Place of search | Date of completion of the search | Examiner |
|---|---|---|
| Munich | 7 July 2022 | Kania, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

Application Number

EP 22 15 3142

## DOCUMENTS CONSIDERED TO BE RELEVANT

| Category | Citation of document with indication, where appropriate, of relevant passages | Relevant to claim |
|---|---|---|
| A,D | Gao Xiquan ET AL: "222 / Molecular Plant-Microbe Interactions Inactivation of the Lipoxygenase ZmLOX3 Increases Susceptibility of Maize to Aspergillus spp", <br> , <br> 1 January 2009 (2009-01-01), pages 222-231, XP055819804, <br> Retrieved from the Internet: <br> URL:https://apsjournals.apsnet.org/doi/abs /10.1094/MPMI-22-2-0222?url_ver=Z39.88-200 3&rfr_id=ori:rid:crossref.org&rfr_dat=cr_p ub%20%200pubmed <br> [retrieved on 2021-06-30] <br> ----- | 1-15 |
| A | CN 110 684 796 A (UNIV FUJIAN AGRICULTURE & FORESTRY) 14 January 2020 (2020-01-14) <br> ----- | 1-15 |

CLASSIFICATION OF THE APPLICATION (IPC)

TECHNICAL FIELDS SEARCHED (IPC)

The present search report has been drawn up for all claims

| Place of search | Date of completion of the search | Examiner |
|---|---|---|
| Munich | 7 July 2022 | Kania, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 3142

This annex lists the patent family members relating to the patent documents cited in the above-mentioned European search report.
The members are as contained in the European Patent Office EDP file on
The European Patent Office is in no way liable for these particulars which are merely given for the purpose of information.

07-07-2022

| Patent document cited in search report | | Publication date | Patent family member(s) | Publication date |
|---|---|---|---|---|
| WO 2021207148 | A1 | 14-10-2021 | NONE | |
| CN 110684796 | A | 14-01-2020 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021170785 A

**Non-patent literature cited in the description**

- **GAO et al.** *MPMI,* 2007, vol. 20 (8), 922-933
- **GAO et al.** *MPMI,* 2008, vol. 21 (1), 98-109
- **LOUTRE et al.** *The Plant Journal,* 2009, vol. 60, 1043-1054
- **KIM et al.** *Nat. Biotech.,* 2017
- **GAO et al.** *BioRxiv*
- **SMITH, T.F. ; WATERMAN, M.S.** Identification of common molecular subsequences. *Journal of Molecular Biology,* 1981, vol. 147 (1), 195-197
- **ZMUBI ; CHRISTENSEN, A.H. ; QUAIL, P.H.** Ubiquitin promoter-based vectors for high-level expression of selectable and/or screenable marker genes in monocotyledonous plants. *Transgenic Research,* 1996, vol. 5, 213-218, https://doi.org/10.1007/BF01969712
- **HELENIUS et al.** Gene delivery into intact plants using the HeliosTM Gene Gun. *Plant Molecular Biology Reporter,* 2000, vol. 18 (3), 287-288
- **E. DIEDERICHSEN ; M.D. SACRISTAN.** *Plant Breeding,* 1996, vol. 115, 5-10